(19) Europäisches Patentamt / European Patent Office / Office européen des brevets

(11) **EP 3 977 915 B1**

(12) **EUROPEAN PATENT SPECIFICATION**

(45) Date of publication and mention
of the grant of the patent:
**19.06.2024   Bulletin 2024/25**

(21) Application number: **20199575.0**

(22) Date of filing: **01.10.2020**

(51) International Patent Classification (IPC):
**A61B 3/10** *(2006.01)*      **G01B 9/02091** *(2022.01)*
**G01B 9/02** *(2022.01)*      **G02B 21/00** *(2006.01)*

(52) Cooperative Patent Classification (CPC):
**A61B 3/102; G01B 9/02091**

(54) **OPHTHALMIC CONFOCAL OCT IMAGING SYSTEM**

OPHTHALMISCHES KONFOKALES OKT-BILDGEBUNGSSYSTEM

SYSTÈME D'IMAGERIE OCT CONFOCAL OPHTHALMIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**06.04.2022   Bulletin 2022/14**

(73) Proprietor: **Optos PLC
Dunfermline KY11 8GR (GB)**

(72) Inventors:
• **Varughese Chacko, Lijo
Dunfermline, Scotland, KY11 8GR (GB)**

• **Preciado, Miguel Angel
Dunfermline, Scotland, KY11 8GR (GB)**

(74) Representative: **Hoffmann Eitle
Patent- und Rechtsanwälte PartmbB
Arabellastraße 30
81925 München (DE)**

(56) References cited:
**WO-A1-2014/089504      JP-A- 2009 008 393
US-A1- 2007 096 014      US-A1- 2007 263 226
US-A1- 2017 105 618**

**Description**

[Technical Field]

Example aspects herein generally relate to the field of ophthalmic optical coherence tomography (OCT) imaging systems and, more particularly, a confocal optical coherence tomography, OCT, imaging system for imaging a region of a subject's eye.

[Background]

Most commercially available OCT imaging systems are point-scan OCT imaging systems which can acquire an OCT image by scanning a laser beam laterally across a region of the eye. In a point-scan OCT imaging system, the speed of image acquisition is limited by the laser scanner and the spot size of the illuminating beam. However, the speed of OCT data acquisition is an important performance metric in OCT, since a low acquisition rate during an OCT scan can result in motion artefacts and phase errors in the acquired OCT data. Parallel acquisition OCT imaging systems, such as Full-Field OCT (FF-OCT) or Line-Field OCT (LF-OCT), may offer superior A-scan acquisition rates (up to tens of MHz) by illuminating an area or a line on the sample, rather than scanning a single spot across the eye. Furthermore, as the pixels of an OCT image can be acquired concurrently, parallel acquisition OCT imaging systems can allow the acquisition of phase-stabilised data.

In full-field OCT, a two-dimensional region of the eye is illuminated at the same time and the lateral positions across the region are concurrently captured using a photodetector array such as a high-speed charge-coupled device (CCD) camera. Full-field OCT may be classified as full-field time-domain OCT (FF-TD-OCT) or full-field swept-source OCT (FF-SS-OCT), for example. In FF-TD-OCT, the optical length of the reference arm can be varied during a scan in order to image regions at different depths in the eye. Each frame captured by the high-speed camera in FF-TD-OCT therefore corresponds to a slice of the eye at a respective depth within the eye. In FF-SS-OCT, the sample region is full-field illuminated using a swept light source that emits light whose wavelength varies over time. As the wavelength of the swept light source is swept over a range of optical wavelengths, a spectrogram correlating reflectivity information against optical wavelength can be generated by the high-speed camera for each camera pixel. Each frame captured by the camera therefore corresponds to reflectivity information for a single wavelength of the swept light source. Upon acquiring a frame for every wavelength of the swept light source, a C-scan of the region can be obtained by performing a Fourier transform on the samples of spectrograms generated by the camera. In line-field OCT (LF-OCT), a line of illumination may be provided to the sample and a B-scan may be acquired in the imaging process. Line-field OCT may be classified as line-field time-domain OCT (LF-TD-OCT), line-field swept-source OCT (LF-SS-OCT), or line-field spectral-domain OCT (LF-SD-OCT), for example.

WO 2014/089504 A1 discloses a parallel imaging optical coherence tomography system, which includes a light source, a movable scan unit, and an interferometer comprising a reference arm and a sample arm. The scan unit includes a plurality of apertures illuminated by incident light from the light source. The scan unit produces a plurality of light beams transmitted to the interferometer. The light beams are each split producing a plurality of reference and sampling beams. The sampling beams are scanned onto a sample and return reflected sampling light signals through the sample arm, which are combined with reflected, reference light signals from the reference arm. An interference pattern is formed, which Is detected by an image sensor and processed to generate a digital image of the sample. The scan unit may include a microlens array.

JP 2009 008393 A discloses an optical image measuring device capable of observing a tomogram of an observation target object with high resolution and contrast. Scanning is performed with a light beam from a light source corresponding to a pinhole array of an axially rotating Nipkow disk, and the light beam is divided into searching light toward the target object and reference light toward a reference optical path by a beam splitter. The searching light from the target object and the reference light through the reference optical path are synthesized by the beam splitter, to thereby generate interference light. The interference light is detected by a two-dimensional imaging means, and reflection intensity information inside the target object is acquired from an image signal. Since a light beam scanning means by the Nipkow disk and the two-dimensional imaging means are utilized, an interference optical system can be realized simply.

US 2007/263226 A1 discloses a tissue imaging system for examining the medical condition of tissue, which has an illumination optical system comprising a light source having one or more light emitters, beam shaping optics, and polarizing optics. An optical beam splitter directs illumination light to an imaging sub-system, containing a spatial light modulator array. An objective lens images illumination light from the spatial light modulator array to the tissue. An optical detection system images the spatial light modulator to an optical detector array. A controller drives the spatial light modulator to provide time-variable arrangements of on-state pixels. The objective lens operates in a nominally telecentric manner relative to both the spatial light modulator and the tissue. The polarizing optics are independently and iteratively rotated to define variable polarization states relative to the tissue. The modulator pixels optically function like pinholes relative to the illumination light and the image light.

[Summary]

The present invention provides a confocal OCT imaging system according to claim 1, a confocal OCT imaging system according to claim 2. Optional features are set out in the dependent claims.

[Brief Description of the Drawings]

Example embodiments, and background examples that are helpful for understanding the invention, will now be explained in detail, by way of non-limiting example only, with reference to the accompanying figures described below. Like reference numerals appearing in different ones of the figures can denote identical or functionally similar elements, unless indicated otherwise.

Fig. 1 is a schematic illustration of a confocal OCT imaging system according to a background example not forming an embodiment, which is helpful for understanding the invention.

Fig. 2 is a block diagram illustrating an example implementation in programmable signal processing hardware of the tomographic image data generating module described herein.

Fig. 3 is a schematic illustration of a first example implementation of the confocal OCT imaging system of the background example.

Fig. 4 is a schematic illustration of a second example implementation of the confocal OCT imaging system of the background example.

Fig. 5 is a schematic illustration of an example implementation of the confocal OCT imaging system of Fig. 3 as a full-field swept source OCT imaging system, in accordance with an example embodiment.

Fig. 6A is a schematic illustration of an example of a photodetector element array which can be used to detect the interference light in an example embodiment herein.

Fig. 6B is a schematic illustration of the processing of an interferogram to generate an A-scan and tomographic image data in an example embodiment herein.

Fig. 7 is a schematic illustration of an implementation of the confocal OCT imaging system of Fig. 3 as a full-field time-domain OCT imaging system, in accordance with a background example not forming an embodiment.

Fig. 8 is a schematic illustration of an example implementation of the confocal OCT imaging system of Fig. 3 as a line-field swept-source OCT imaging system, in accordance with an example embodiment.

Fig. 9 is an example illustration of a line crossing the region of the eye that is scanned by the line-field swept source OCT imaging system of Fig. 8.

Fig. 10 is a schematic illustration of an implementation of the confocal OCT imaging system of Fig. 3 as a full-field time-domain OCT imaging system, in accordance with a background example not forming an embodiment.

Fig. 11 is a schematic illustration of an implementation of the confocal OCT imaging system of Fig. 3 as a line-field spectral domain OCT imaging system, in accordance with a background example not forming an embodiment.

[Detailed Description of Example Embodiments]

A disadvantage of parallel acquisition OCT imaging systems of the kinds set out above is they lack the confocal gating of point-scan OCT imaging systems. This can cause unwanted reflections and cross-talk between light from different lateral locations of the eye during a capture by the high-speed 2D camera. In point-scan OCT imaging systems, the cross-talk tends to be avoided or suppressed as a confocal pinhole is typically placed directly in front of the photodetector to reject out-of-focus light and any light scattered by different lateral positions of the eye. However, as the two-dimensional region is simultaneously recorded by the camera in full-field OCT, confocality is lost in both in the lateral scanning direction as well as the axial scanning direction (i.e. depth direction) of the eye. With line-field OCT, a degree of confocality is achieved through the use of line illumination. However, a very high numerical aperture is required to produce a confocal gate that can prevent most scattered light from interfering with the desired signal.

There is described herein, in accordance with a first example aspect herein which does not form an embodiment of the invention but is nevertheless helpful for understanding the invention, a confocal OCT imaging system for imaging a region of a subject's eye. The confocal OCT imaging system comprises a light source arranged to generate light, and an illumination module comprising a mask having a plurality of transmissive portions through which the light from the light source can propagate to form respective non-overlapping beams of light when the mask is illuminated by the light source, the illumination module being arranged to perform a scan of the region of the subject's eye by scanning the plurality of non-overlapping beams across the region such that each point in the region is illuminated by a respective one of the plurality of non-overlapping beams during the scan.

The confocal OCT imaging system further comprises an interferometer comprising a beam splitter arranged to generate reference light based on the light generated by the light source, the interferometer being arranged to generate interference light by combining, for each beam, light from the beam which has been reflected from the region with the reference light generated by the beam splitter. The confocal OCT imaging system further comprises a photodetector element array arranged to detect the interference light generated during the scan, and a tomographic image data generating module arranged to generate tomographic image data defining a tomographic image of the region based on the detected interference light.

In the confocal OCT imaging system of the first example aspect, the interferometer may, in accordance with a first example implementation, have a reference arm comprising a reference mirror, and the beam splitter may be arranged to extract light from each of the beams and direct the extracted light to the reference mirror, wherein the mirror is arranged to reflect the extracted light back to the beam splitter as the reference light, and wherein the beam splitter is arranged to generate the interference light by combining, for each beam, light from the beam which has been reflected from the region with the light reflected back to the beam splitter by the mirror.

In the confocal OCT imaging system of the first example aspect, the interferometer may, in accordance with a second example implementation, comprise a second beam splitter and a reference arm along which the reference light is arranged to propagate from the beam splitter to the second beam splitter, the second beam splitter being arranged to generate the interference light by combining, for each beam, light from the beam which has been reflected from the region with the reference light propagating along the reference arm.

In the confocal OCT imaging system of the first example aspect or any of the example implementations set out above, the mask may, in accordance with a third example implementation, be further arranged to allow the light which has been reflected from the region of the subject's eye to propagate to the photodetector element array only through the transmissive portions of the mask.

In the confocal OCT imaging system of the first example aspect or any of the example implementations set out above, the photodetector elements may, in accordance with a fourth example implementation, have an integration time smaller than a duration of the scan and are arranged to make concurrent measurements of the interference light in each of a plurality of integration time intervals during the scan, and the tomographic image data generating module may be arranged to combine the measurements made by the photodetector elements in each of the integration time intervals so as to generate a combined measurement for the scan.

In the confocal OCT imaging system of the first example aspect or any of the first to third example implementations set out above, each of the photodetector elements may, in accordance with a fifth example implementation, have an integration time substantially equal to a duration of the scan and is arranged to make a respective single measurement of the interference light during the scan, and the tomographic image data generating module may be arranged to generate the tomographic image data based on the measurements made by the photodetector elements.

In the confocal OCT imaging system of the first example aspect or any of the example implementations set out above, the ophthalmic OCT imaging system may, in accordance with a sixth example implementation, be a swept-source OCT, SS-OCT, imaging system, and the light source may be a swept light source arranged to generate light of varying wavelength, wherein the illumination module is arranged to perform a plurality of scans of the region of the subject's eye, while the wavelength of the light is being varied, by scanning the plurality of non-overlapping beams across the region such that each point in the region is illuminated by a respective one of the plurality of non-overlapping beams during each of the scans, and wherein the photodetector elements are arranged to detect the interference light generated during each of the scans.

In the confocal OCT imaging system of the first example aspect or any of the first to fifth example implementations set out above, the interferometer may, in accordance with a seventh example implementation, comprise a reference arm along which the reference light generated by the beam splitter propagates, and the ophthalmic OCT imaging system may be a time-domain OCT, TD-OCT, imaging system comprising an optical path adjustment mechanism arranged to vary an optical path length of the reference arm, wherein the illumination module is arranged to perform a plurality of scans of the region of the subject's eye, while the optical path length is being varied by the optical path adjustment mechanism, by scanning the plurality of non-overlapping beams across the region such that each point in the region is illuminated by a respective one of the plurality of non-overlapping beams during each of the scans, and wherein the photodetector elements are arranged to detect the interference light generated during each of the scans.

In the confocal OCT imaging system of the first example aspect or any of the example implementations set out above, the light source may, in accordance with a eighth example implementations, be a full-field OCT light source arranged to provide full-field illumination to the illumination module.

In the confocal OCT imaging system of the first example aspect or any of the first to seventh example implementations set out above, the light source may, in accordance with a ninth example implementation, be a line-field OCT light source arranged to provide a line of light to the illumination module, and the illumination module may be arranged to perform a scan of the region of the subject's eye by scanning the plurality of non-overlapping beams across each of a plurality of lines crossing the region such that each point in the region is illuminated by a respective one of the plurality of non-overlapping beams during the scan.

In the confocal OCT imaging system of the first example aspect or any of the first to seventh example implementations set out above, the ophthalmic OCT imaging system may, in accordance with a tenth example implementation, be a spectral-domain OCT, SD-OCT, imaging system, and the light source may be a broadband line-field OCT light source arranged to provide a line of the light to the illumination module. The illumination module may be arranged to perform a scan of the region of the subject's eye by scanning the plurality of non-overlapping beams across each of a plurality of lines crossing the region such that each point in the region is illuminated by a respective one of the plurality of non-overlapping beams during the scan, and the SD-OCT imaging system may further comprise a dispersive element arranged to spatially separate frequency components of the interference light, wherein the photodetector elements are arranged to detect respective frequency components of the interference light that have been spatially separated by the dispersive element, and the tomographic image data generating module is arranged to generate the tomographic image data by calculating an inverse Fourier transform of the detected frequency components.

In the confocal OCT imaging system of the first example aspect or any of the example implementations set out above, the mask may, in accordance with an eleventh example implementation, be arranged to rotate during the scan, and the transmissive portions may be distributed across the mask such that each point in the region is illuminated by a respective one of the plurality of non-overlapping beams as the mask rotates during the scan. The plurality of transmissive portions may be distributed across the mask in an Archimedean spiral. Furthermore, the illumination module may further comprise a plurality of lenslets, each of the lenslets being arranged to direct light from the light source through a respective one of the transmissive portions in the mask, and the plurality of lenslets may be arranged to rotate together with the mask so as to maintain an alignment of each lenslet with the respective transmissive portion during rotation of the mask and the plurality of lenslets. Furthermore, the transmissive portions of the mask may comprise at least one of pinholes or slits in the mask.

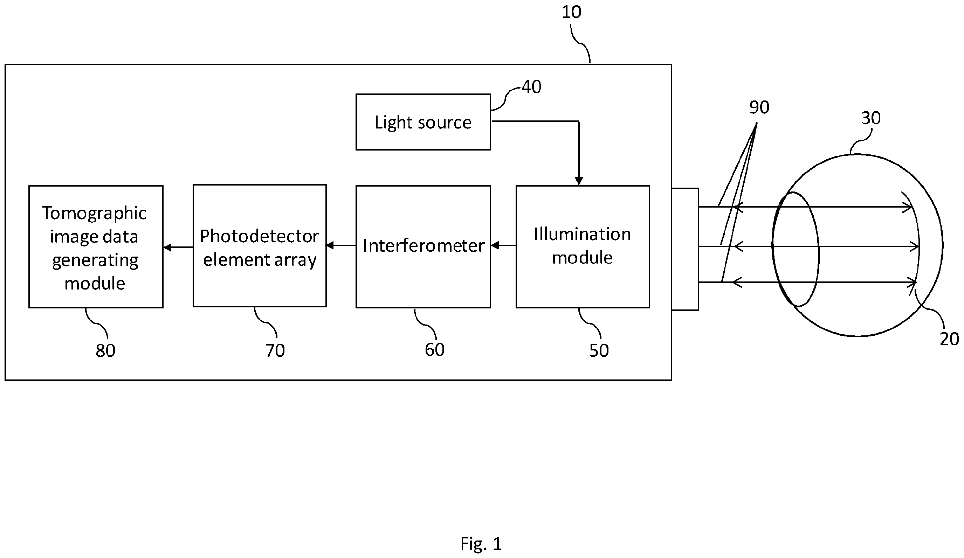

Figure 1 is a schematic illustration of an OCT imaging system 10 for imaging a region 20 of a subject's eye 30, according to a first background example.

The confocal OCT imaging system 10 comprises a light source 40 arranged to generate light, an illumination module 50, an interferometer 60, a photodetector element array 70, and a tomographic image data generating module 80.

The illumination module 50 comprises a mask having a plurality of transmissive portions through which the light from the light source 40 can propagate to form respective non-overlapping beams 90 of light when the mask is illuminated by the light source 40. The illumination module 50 is arranged to perform a scan of the region of the subject's eye 30 by scanning the plurality of non-overlapping beams 90 across the region 20 such that each point in the region 20 is illuminated at least once by a respective one of the non-overlapping beams 90 during the scan.

The interferometer 60 comprises a beam splitter that is arranged to generate reference light based on the light generated by the light source 40. Furthermore, the interferometer is arranged to generate interference light by combining, for each beam, light from the beam which has been reflected from the region with the reference light generated by the beam splitter.

The photodetector element array 70 is arranged to detect the interference light generated during the scan. Furthermore, the tomographic image data generating module 80 is arranged to generate tomographic image data defining a tomographic image of the region 20 based on the detected interference light.

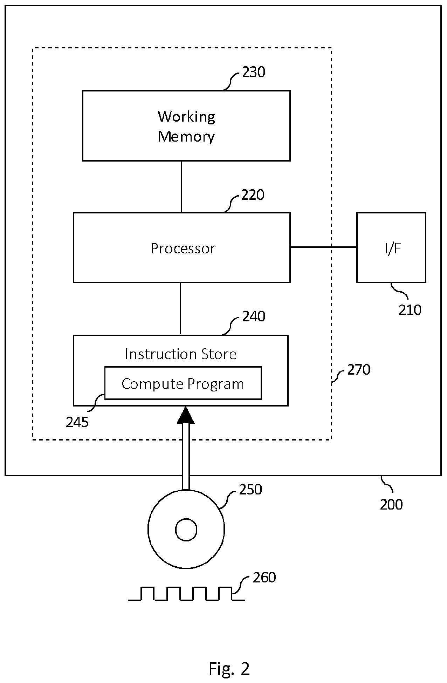

Fig. 2 shows an example implementation of the tomographic image data generating module 80 of Fig. 1, in the form of programmable signal processing hardware. In one example embodiment herein, the signal processing apparatus 200 can form the tomographic image data generating module of Fig. 1. The signal processing apparatus 200 comprises an interface module 210 for receiving measurements made by the photodetector element array 70. The signal processing apparatus 200 further comprises a processor (CPU) 220, a working memory 230 (e.g. a random-access memory) and an instruction store 240 storing a computer program 245 comprising computer-readable instructions which, when executed by the processor 220, cause the processor 220 to perform the processing operations of the tomographic image data generating module 80. The instruction store 240 may comprise a ROM (e.g. in the form of an electrically-erasable programmable read-only memory (EEPROM) or flash memory) which is pre-loaded with the computer-readable instructions. Alternatively, the instruction store 240 may comprise a RAM or similar type of memory, and the computer-readable instructions can be input thereto from a computer program product, such as a computer-readable storage medium 250 such as a CD-ROM, etc. or a computer-readable signal 260 carrying the computer-readable instructions. The combination of the hardware components shown in Fig. 2, comprising the processor 220, the working memory 230 and the instruction store 240, is configured to perform functions of the tomographic image data generating module 80.

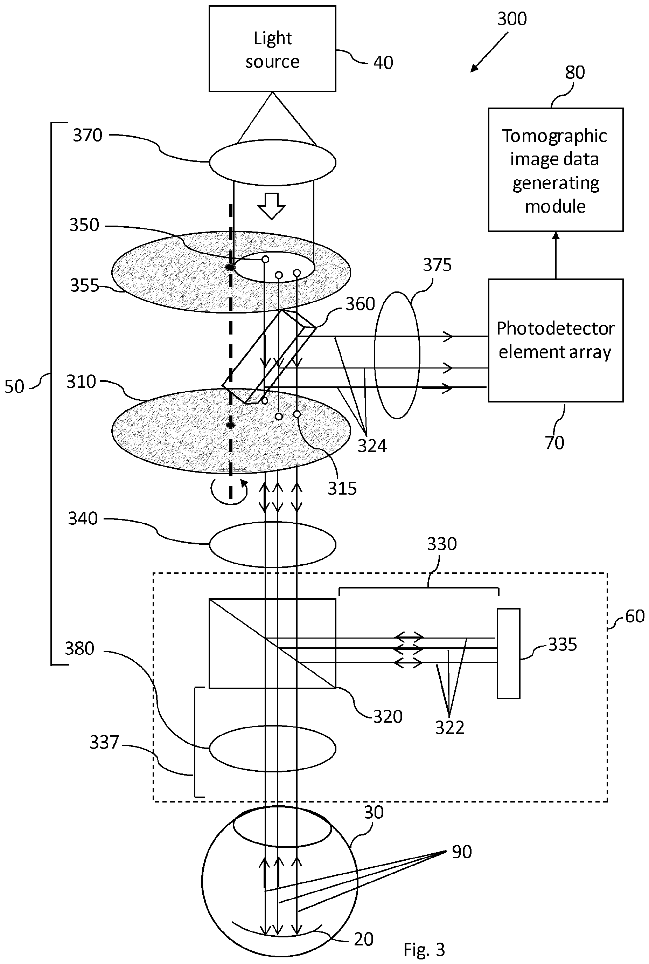

Figure 3 illustrates an example implementation of the confocal OCT imaging system 10 of Fig. 1. The illumination module 50 of the confocal OCT imaging system of Fig. 3 comprises a mask 310, which may take the general form of a sheet of opaque material having a plurality of light-transmissive portions 315 distributed across its surface. For clarity of illustration, only the light-transmissive portions 315 in a portion of the mask 310 that is illuminated by the light source 40 are shown in Fig. 3. By way of an example, the mask 310 illustrated in Fig. 3 is an aluminium disc having transmissive portions 315 in the example form of a plurality of pinholes in the disc. However, it is noted that the shape of the mask 310 is not so limited, and the mask 310 may be formed of a metal other than aluminium or, more generally, any other opaque material (e.g. a plastic such as polyaryletheretherketone, PEEK). Furthermore, the transmissive portions 315 of the mask 310 are not limited to pinholes and may alternatively be provided in the form of slits or any other form of small openings that allow light to pass through the mask 310 (or, more generally, portions of the mask that are transparent and small enough to provide a confocal gating of light as discussed above). The size of the transmissive portions 315 may be selected based on required level of confocal gating for the OCT imaging system.

The illumination module 50 is arranged to perform a scan of the region 20 of the subject's eye 30 by scanning the plurality of non-overlapping beams 90 formed by the mask 310 across the region 20 of the eye 30 such that each point in the region 20 is illuminated by a respective one of the non-overlapping beams 90 during the scan. In the present example implementation, the region 20 comprises a part of a retina of the eye 30, but may alternatively comprise another part of the eye 30, such as an anterior region of the eye 30, for example.

The illumination module 50 may be arranged in one of a number of different ways to scan the non-overlapping beams 90 across the region 20 so that each point in the region 20 is illuminated by a respective one of the non-overlapping beams 90 during the scan. By way of an example, the illumination module 50 of the present background example is arranged to perform the scan of the region 20 by rotating the mask 310 during the scan, specifically by rotating the disc with the pinholes (310) shown in Fig. 3 about its rotational axis of symmetry, as illustrated in Fig. 3. The transmissive portions 315 are distributed across the mask 310 such that each point in the region 20 is illuminated by a respective one of the plurality of non-overlapping beams 90 as the mask 310 rotates during the scan. More specifically, in the present background example, the transmissive portions 315 rotate with the mask 310, thereby causing the plurality of non-overlapping beams 90 emerging from the mask 310 to be scanned in parallel across the region 20 of the eye 30, via a collimating lens 340 and a focusing lens 380 that also form part of the illumination module 50, as illustrated in Fig. 3.

The transmissive portions 315 may, as in the present background example, be distributed across the mask 310 in a predetermined pattern, for example in an Archimedean spiral. However, it should be noted that the transmissive portions 315 need not be arranged in the shape of an Archimedean spiral or according to another predetermined pattern, and may alternatively be randomly distributed across the mask 310. It should also be noted that the illumination module 50 need not be arranged to perform the scan of the region 20 by rotating the mask 310 during the scan but may alternatively comprise a stationary mask 310 and an optical arrangement including, for example, a moving mirror that causes the each point in the region 20 to be illuminated by a respective one of the plurality of non-overlapping beams 90 as the mirror rotates during the scan.

The interferometer 60 may take one of a number of different forms known to those versed in the art, and the form of the interferometer 60 is not limited to any of the examples described herein. In the present background example, the interferometer 60 comprises a beam splitter 320 that is arranged to generate reference light 322 based on the light generated by the light source 40, as illustrated in Fig. 3. Furthermore, the interferometer 60 is arranged to generate interference light 324 by combining, for each beam 90, light from the beam which has been reflected from the region 20 of the eye 30 with the reference light 322 generated by the beam splitter 320.

The interferometer 60 may, as shown in Fig. 3, have a double-pass reference arm 330 that comprises a reference mirror 335 at one of its ends, and a sample arm 337 extending from the beam splitter 320 to the eye 30. The beam splitter 320 is arranged to extract light from each of the plurality of non-overlapping beams 90 and direct the extracted light to the reference mirror 335, the reference mirror 335 being arranged to reflect the extracted light back to the beam splitter 320 as the reference light 322. The beam splitter 320 thus receives the plurality of non-overlapping beams 90 propagating from the mask 310 and directs a first portion of the light from the plurality of non-overlapping beams 90 towards the reference mirror 335, and directs a second portion of the light from the plurality of non-overlapping beams 90 towards the region 20 of the eye 30. Furthermore, the beam splitter 320 is arranged to generate the interference light 324 by combining, for each beam 90, light from the beam which has been reflected from the region 20 with the light reflected back to the beam splitter 320 by the mirror 335.

In Fig. 3, the photodetector element array 70 is arranged to detect the interference light 324 generated during the scan. In the present background example, where the mask 310 is rotated to scan the plurality of non-overlapping beams 90, the interference light 324 is generated by the beam splitter 320 combining the reference light 322 with light reflected from the areas of the region 20 of the eye 30 that are scanned by the plurality of non-overlapping beams 90 as the mask 310 is rotated. The photodetector element array 70 may, as in the present background example, form part of a CCD camera, but may alternatively from part of a CMOS sensor or any other light detector having a two-dimensional photodetector element array. The tomographic image data generating module 80 is arranged to generate tomographic image data defining a tomographic image of the region 20 based on the interference light 324.

The illumination module 50 may, as in the present background example, further comprise a plurality of lenslets 350, each lenslet arranged to direct light from the light source 40 through a respective one of the transmissive portions 315 of the mask 310. Furthermore, where the mask 310 is arranged to rotate during a scan, the plurality of lenslets 350 is arranged to rotate together with the mask 310 so as to maintain an alignment of each lenslet 350 with the respective one of the transmissive portions 315 during rotation of the mask 310 and the plurality of lenslets 350. The plurality of lenslets 350 may, as in the present background example, be mounted on a lenslet disc 355 that is also included in the illumination module 50 and arranged to rotate in synchronisation with the mask 310. Furthermore, the mask 310 may, as in the present background example, be positioned in a focal plane of the plurality of lenslets 350 such that each of the lenslets 350 focuses light from the light source 40 onto a respective one of the transmissive portions 315 of the mask 310. By using a plurality lenslets 315 to focus light from the light source 40 onto the transmission portions 315 of the mask 310, the fill-factor (defined as the proportion of the light incident on the mask 310 that is transmitted through the mask 310) can be significantly increased. On the other hand, in examples not having the lenslets 350, most of the light from the light source 40 may be blocked by the mask 310.

In the confocal OCT imaging system 300 of Fig. 3, the mask 310 is further arranged to allow the light which has been reflected from the region 20 of the subject's eye 30 to propagate to the photodetector element array 70 only through the transmissive portions 315 of the mask 310, and a second beam splitter 360 is provided to receive the interference light 324 from the beam splitter 320, which has passed through the transmissive portions 315 of the mask 310, and to reflect the interference light 324 to the photodetector element array 70. The interference light 324 may thus be spatially filtered by the mask 310 before it is detected by the photodetector element array 70, thereby providing further level of confocal gating.

It should be noted, however, that although the mask 310 in Fig. 3 modulates both the light that is scanned onto the eye 30 and the light reflected from the eye 30, the mask 310 need not, in general, modulate the light returned from the eye 30 to achieve cross-talk rejection. For example, in an alternative example, the confocal OCT imaging system 300 may not have the aforementioned second beam splitter 360, and the beam splitter 320 may instead be arranged to direct the interference light 324 to a photodetector element array 70 positioned on an opposite side of the beam splitter 320 to the reference mirror 335, for example, thus without the interference light 324 passing through the transmissive portions 315 of the mask 310.

As noted above, the illumination module 50 may, as in the present background example, further comprise one or more optical elements for guiding light into the eye 30. For example, as shown in Fig. 3, the illumination module 50 may additionally comprise collimating a lens 340 and a focusing lens 380. Furthermore, the confocal OCT imaging system 300 may also have a collimating lens 370 arranged to collimate light from the light source 40 and provide collimated light to the mask 310. The collimating lens 340 is arranged to collimate the plurality non-overlapping beams propagating from the transmissive portions 315 of the mask 310 and provide a plurality of collimated beams to beam splitter 320. A collimating lens 375 may also be included in the confocal OCT imaging system 300 and arranged to collimate the interference light 324 before it is detected by the photodetector element array 70, as illustrated in Fig. 3. Furthermore, as shown in Fig. 3, the focusing lens 380 is arranged to focus the non-overlapping beams 90 onto a plurality of respective points on the region 20 of the eye 30.

In the present background example, each photodetector element of the photodetector element array 70 may have an integration time substantially equal to a duration of the scan, and is arranged to make a respective single measurement of the interference light 324 during the scan. However, the integration time need not be substantially equal to the duration of the scan.

In some example embodiments, each of the photodetector elements has an integration time which is larger than the duration of the scan such that multiple scans of the region 20 are performed by the illumination module 50 for each measurement made by the photodetector element.

Furthermore, in other background example, the photodetector elements of the array 70 may have an integration time that is smaller than a duration of the scan, and may be arranged to make concurrent measurements of the interference light 324 in each of a plurality of integration time intervals during the scan. In these background examples, the tomographic image data generating module 80 is arranged to combine the measurements made by the photodetector elements in each of the integration time intervals in order to generate a combined measurement for the scan.

The confocal OCT imaging system 300 of Fig. 3 illustrates just one example implementation of the confocal OCT imaging system 10 of Fig. 1. In particular, Fig. 3 illustrates an example implementation comprising an interferometer having a double-pass reference arm, wherein the beam splitter 320 extracts light from the non-overlapping beams 90 propagating from the mask 310 and provides the extracted light to a mirror 335 in the reference arm 335, and wherein light reflected by the mirror 335 is further combined at beam splitter 320 with light reflected from the region 20 of the eye 30 to generate the interference light 324. Accordingly, in Fig. 3, the same beam splitter which generates the reference light 322 also generates the interference light 324.

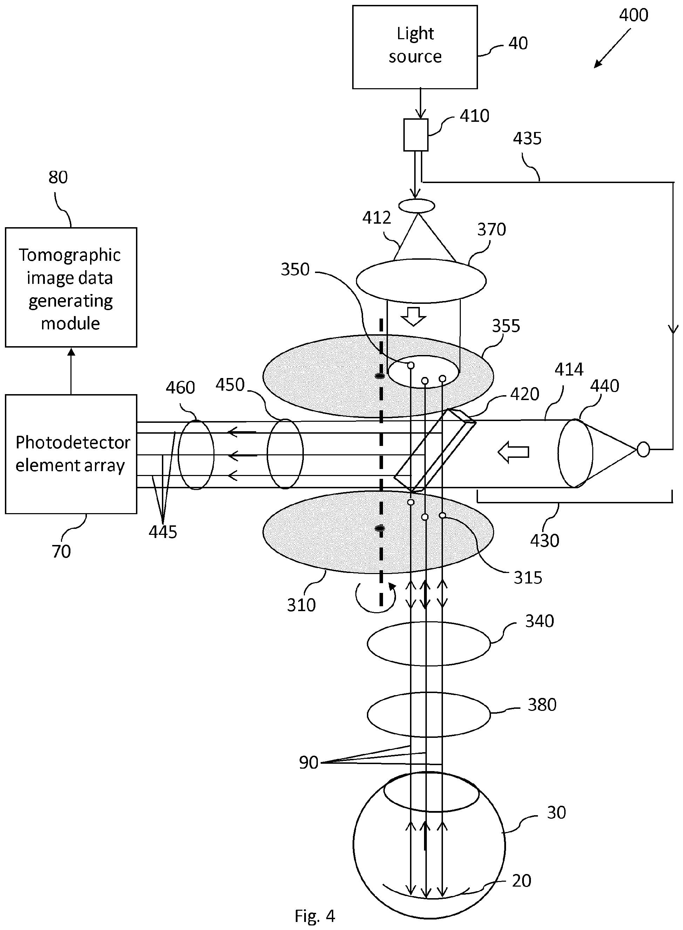

An alternative implementation of the confocal OCT imaging system 10 of Fig. 1, which employs an interferometer having a single-pass reference arm, is illustrated in Fig. 4. In the alternative implementation of Fig. 4, components of the illustrated confocal OCT imaging system 400 that are the same as those in the confocal OCT imaging system 300 described above with reference to Fig. 3 are labelled with like numerals, and the description of these components and their variants, as well as other features (and variants) of the confocal OCT imaging system 300 that are also features (and corresponding variants) of the confocal OCT imaging system 400, will not be described again here. The following description of the alternative implementation of the confocal OCT imaging system 400 shown in Fig. 4 will instead focus on the differences between these implementations.

As shown in Fig. 4, a beam splitter 410 is arranged to split the light from the light source 40 into a sample light 412 that is provided to the illumination module 50 comprising the mask 310, and a reference light 414 that is provided to a reference arm 430. The reference arm 430 may, as shown in Fig. 4, comprise a span of optical fiber 435 that directs the reference light 414 from beam splitter 410 to a collimating lens 440 of the reference arm, which is arranged to collimate the reference light 414 and provide the collimated reference light 414 to a beam splitter 420. It should be noted the confocal OCT imaging system 400 is not limited to the fiber optic implementation illustrated in Fig. 4. For example, the reference arm may alternatively be implemented entirely in free space by using one or more mirrors to guide the reference light 414 from the beam splitter 410 to beam splitter 420. As another example, some one or more portions of the reference arm 430 may take this free-space implementation, while light in the remainder of the reference arm 430 is guided by one or more spans of optical fiber. Furthermore, although Fig. 4 illustrates the beam splitter 410 to be a fiber optic coupler, the beam splitter 420 may alternatively be half-silvered mirror or a dichroic mirror, for example.

In the implementation of Fig. 4, a beam splitter 420 is provided between the lenslet disc 355 and the mask 310 (similar to the beam splitter 360 in Fig. 3), and arranged to generate interference light 445 by combining, for each beam of the plurality of non-overlapping beams 90, light from the beam which has been reflected from the region 20 of the eye 30 with the reference light 414, which propagates from the beam splitter 410 along the optical fiber 435 and via the collimating lens 440. It should be noted, however, that although the light which has been reflected from the region 20 of the eye 30 has passed through the transmissive portions 315 of the mask 310 after being reflected from the region 20, the confocal OCT imaging system 400 is not limited in this aspect, and the beam splitter 420 may instead be arranged to generate the interference light 445 on the basis of light which has been reflected from the region 20 but has not passed through the transmissive portions 315 of the mask 310 after being reflected from the region 20. For example, in Fig. 4, beam splitter 420 may alternatively be situated on the sample (lower) side of mask 310 rather than the light source (upper) side.

In Fig. 4, beam splitter 420 is further arranged to provide the interference light 445 to the photodetector element array 70. The interference light 445 may, as shown in Fig. 4, be provided to the photodetector element array 70 via a collimating lens 450 that is arranged to collimate the interference light 445 from beam splitter 420, and a focusing lens 460 that is arranged to focus the interference light 445 onto the photodetector element array 70.

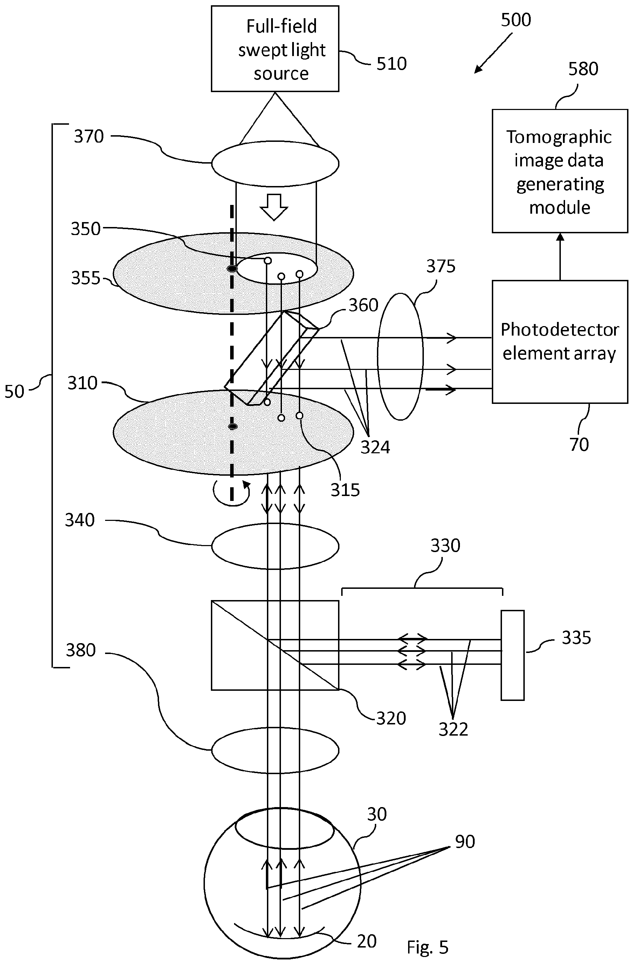

Figure 5 illustrates an example implementation of the confocal OCT imaging system 300 of Fig. 3 as a full-field, swept-source OCT (FF-SS-OCT) imaging system. It should be noted that, although the following example implementations are described in relation to confocal OCT imaging system 300 of Fig. 3, the described principles and implementation details are also applicable the confocal OCT imaging system 400 of Fig. 4, and to any of the variants of these confocal OCT imaging systems described with reference to Figs. 3 and 4.

The FF-SS-OCT imaging system 500 illustrated in Fig. 5 comprises all of the components of the confocal OCT imaging system 300 in Fig. 3, with the light source 40 being provided in the form of a swept light source 510 which is arranged to generate light of a varying wavelength. Furthermore, the swept-light source 510 is full-field light source, which is arranged to illuminate a two-dimensional region of the illumination module 50. The swept light source 510 may, as in the present example embodiment, be arranged to output substantially monochromatic light while the wavelength of the monochromatic light is swept across a plurality of wavelength values. For example, denoting the wavenumber of the light output by the swept light source 510 at time $t$ by $k(t)$, the wavenumber $k(t)$ may be swept linearly and can be written as $k(t) = k_0 + \delta k \times t$, wherein $k_0$ is a starting wavenumber at the start of the sweep, and $\delta k = \Delta k/\Delta t$ is a rate at which the wavenumber of the output light is swept, wherein $\Delta k$ is the range over which the wavenumber changes during the sweep, and $\Delta t$ is the duration of the sweep. The linewidth of the swept light source 510 (i.e. a width, such as a full width at half-maximum (FWHM), of the frequency spectrum of light generated by the swept light source 510) determines the coherence length of the light and therefore the imaging depth of the FF-SS-OCT imaging system 500, while the wavelength/wavenumber sweep range determines axial resolution.

In Fig. 5, the illumination module 50 is arranged to perform a plurality of scans of the region 20 of the subject's eye 30, while the wavelength of the light is being varied, by scanning the plurality of non-overlapping beams 90 across the region 70 such that each point in the region 70 is illuminated by a respective one of the plurality of non-overlapping beams 90 during each of the scans. The sweep rate ($\delta k$) of the swept-light source 510 and the duration of each scan are such that any variation in the wavelength of the light generated by the swept-light source 510 on the time-scale of each scan is negligible. Consequently, the illumination module 50 may, as in the present example embodiment, perform a scan of the region 20 using light of each wavelength of a plurality of different wavelengths of light output by the swept light source 510. Each scan may therefore be performed using light of a single wavelength of the plurality of wavelengths output by the swept light source 510. Moreover, the photodetector element array 70 is arranged to detect the interference light 324 generated during each of the scans.

The mask 310 of the FF-SS-OCT imaging system 500 of Fig. 5 is arranged to rotate with an angular velocity that is dependent on sweep rate of the swept light source 510, wherein the sweep rate is defined as a rate at which the swept light source 510 varies the wavelength of its output light across a range of wavelength values. The mask 310 is arranged to rotate at an angular velocity such that, for each wavelength of the plurality of wavelengths of light output by the swept light source 510, each point in the region 20 of the eye 30 is illuminated by a respective one of the plurality of non-overlapping beams 90 of the wavelength at least once. In other words, the mask 310 is rotated at a rate such that the entire area of the region 20 is scanned using illumination light of each wavelength of the plurality of wavelengths of light output by the swept light source 510.

The mask 310 may, as the present example embodiment, be rotated for a predetermined number of revolutions such that the entire area of the region 20 is imaged using light of each wavelength of the plurality of wavelengths. For example, if N revolutions of the mask 310 are required to scan the plurality of non-overlapping beams 90 across the entire area of the region 20, and the range of the frequency sweep of the swept light source is B Hz, then a total of at least BN revolutions are required to illuminate the region 20 using light of each frequency within the range of the frequency sweep. By using a mask having a higher density of transmissive portions, fewer revolutions may be required to scan the plurality of non-overlapping beams 90 across the entire region 20. Furthermore, by increasing the rotational speed of the mask 310, the duration of each scan can be reduced, as the plurality of non-overlapping beams 90 can be scanned across the entire area of the region 20 at a higher rate, thereby allowing for a faster image acquisition.

In the FF-SS-OCT imaging system 500 in Fig. 5, the photodetector element array 70 detects the interference light 324 resulting from the illumination of the entire area of the region 20 by light of each wavelength of the plurality of wavelengths of the swept light source 510. In this regard, each photodetector element in the array 70 may, as in the present example embodiment, have an integration time substantially equal to a duration of each scan, and be arranged to make a respective single measurement of the interference light during each scan. The tomographic image data generating module 580 is arranged to generate the tomographic image data based on the measurements made by the photodetector element array 70 over the plurality of scans. For the FF-SS-OCT imaging system 500 of the present example embodiment, the duration of a scan is the time required to scan the plurality of non-overlapping beams 90 of a single wavelength (of the plurality of wavelengths) across the region 20 such that each point in the region 20 is illuminated at least once by a respective one of the plurality of non-overlapping beams 90 of the single wavelength. The measurement made by each photodetector element of the array 70 during each scan is therefore based on interference light signal 324 detected by the photodetector element when the region 20 is scanned by illumination light (i.e. the plurality of non-overlapping beams 90) of the single wavelength. Each photodetector element of the array 70 may, as in the present example embodiment, be illuminated for a brief period of time during each integration time, and a single measurement may be acquired by each of the photodetector elements after the entire area of the region 20 has been illuminated using light of the single wavelength during the scan.

As the wavelength of the swept light source 510 is varied across its plurality of wavelength values, each photodetector element may, as in the FF-SS-OCT imaging system 500 of the present example embodiment, obtain a plurality of measurements that form an interferogram correlating measured intensity against wavelength. Upon each photodetector element obtaining a plurality of measurements corresponding to the plurality of respective wavelengths of light, the tomographic image data generating module 580 may, as in the present example embodiment, generate tomographic image data representing an image of the region 20 by using the plurality of measurements made by each photodetector element.

It should be noted that the illumination module 50 does not perform a single scan of the region 20 for each wavelength of light output by the swept light source 510 in some example embodiments. As the wavelength of the light output by the swept light source 510 is swept across the plurality of wavelength values, the illumination module 50 is arranged to perform a plurality of scans for each wavelength of light, wherein, for each scan, the entire area of the region 20 of the eye 30 is illuminated by the plurality of non-overlapping beams 90 of the wavelength as the mask 310 is rotated. Furthermore, each photodetector element of the array 70 is arranged to generate, for each wavelength, a plurality of measurements of the interference light 324 corresponding to the plurality of scans performed using light of that wavelength. The tomographic image data generating module 580 further performs, for each photodetector element, and for each wavelength, a determination of an averaged measurement by averaging the plurality of measurements obtained by the photodetector element for the wavelength. The tomographic image data generating module 580 further generates tomographic image data based on the averaged measurement for each photodetector and for each wavelength. The number of scans performed for each wavelength can be increased by increasing the rotation speed of the mask 310 and/or by using a mask 310 having a higher density of transmissive portions 315. By performing multiple scans for each wavelength of light and averaging multiple photodetector measurements for each wavelength, the signal-to-noise ratio (SNR) of the resulting tomographic image can be improved as any SNR degradation that would be caused by cross-talk is reduced through the averaging process.

It is also noted that the integration time of the photodetector elements is not substantially equal to the duration of the scan in some example embodiments.

In some example embodiments, the integration time of each photodetector element is longer than the duration of the scan, such that multiple scans of the region 20 can be performed using illumination light of the same wavelength before a measurement is acquired by the photodetector element. In such example embodiments, each point of the region of the eye may be illuminated multiple times by the illumination module 50 during each integration time period. During a single integration time period, if the pattern of illumination on the region 20 changes each time the same spot of the region 20 is illuminated, the lateral interferences would be different each time the spot is illuminated, thereby further reducing lateral interference.

In some other background examples, the photodetector elements of the array 70 may have an integration time smaller than a duration of the scan and may be arranged to make concurrent measurements of the interference light in each of a plurality of integration time intervals during the scan. Furthermore, the tomographic image data generating module 580 of such background examples is arranged to combine the measurements made by the photodetector elements in each of the integration time intervals in order to generate a combined measurement for region 20. For example, in the present FF-SS-OCT imaging system 500, each scan is performed by scanning the plurality of non-overlapping beams 90 of a single wavelength across the region 20 such that each point in the region 20 is illuminated by a respective one of the plurality of non-overlapping beams 90 during the scan. Therefore, in the case where the entire area of the region 20 is illuminated only once in each scan (that is, illuminated only once using light of each wavelength), when the integration time is smaller than the duration of the scan, the interference light 324 measured by the photodetector element array 70 in each of the plurality of integration time intervals is the result of light reflected from the eye 30 when the plurality of non-overlapping beams 90 of light of the single wavelength is scanned over only a portion of the region 20. In this case, a complete measurement of the entire area of the region 20 for a single wavelength is provided by combining the measurements acquired by the photodetector element array 70 over the plurality of integration time intervals during the scan.

In general, for the FF-SS-OCT imaging system 500 of the present example embodiment, the speed of the OCT image acquisition depends on factors such as the fill factor, the rotational speed of the mask 310, the sweep rate of the swept source 510 and the integration time of the photodetector element array 70. Although the present FF-SS-OCT imaging system 500 may provide a lower image acquisition rate than a conventional full-field OCT imaging system, it is nevertheless capable of providing confocality, since the mask 310 with its transmissive portions 315 can prevent light from different lateral locations of the eye 30 from interfering. Furthermore, by rotating the mask 310 to scan the plurality of non-overlapping beams 90 across the region 20, a higher image acquisition rate may be achieved as compared to conventional point-scan OCT imaging systems.

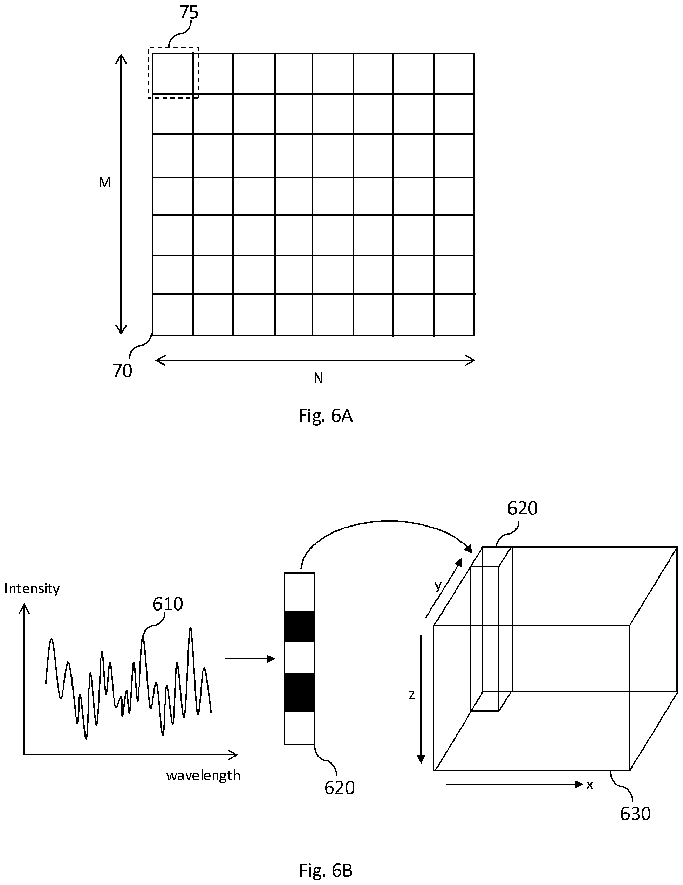

Figure 6A illustrates an example of a photodetector element array 70, which can be used to detect the interference light 324 in accordance with an example embodiment. In Fig. 6A, photodetector elements 75 are arranged in an $M \times N$-sized photodetector element array 70 having $M$ rows and $N$ columns of photodetector elements 75, wherein each photodetector element 75 is identified by a respective location in the array 70 identified by coordinates $(i, j)$, where $i = 0, 1, ... M - 1$, and $j = 0, 1, ... N - 1$. Each photodetector element 75 may, as in the present example embodiment, be mapped to a corresponding location in the region 20 of the eye 30, such that the interference light 324 detected by the photodetector element 75 substantially corresponds to light reflected from the corresponding location.

For the FF-SS-OCT imaging system 500 shown in Fig. 5, each photodetector element 75 of the photodetector element array 70 in Fig. 6 is arranged to output an interferogram comprising the plurality of measurements that are made for the respective plurality of wavelengths of the swept light source 510. More specifically, the interferogram is generated by a photodetector element 75 based on the intensity of the interference light 324 detected by the photodetector element 75 as the wavelength of the swept light source 510 is varied across its plurality of wavelength values. As an example, for a region 20 of the eye 30 having $N$ retinal layers, each layer having a depth $z_n$ from the surface the retina, a measurement $I_{ij}(k)$ (corresponding to a value of the interferogram for a particular wavelength) generated by the photodetector element 75 located at pixel location $(i, j)$, for wavenumber $k$ is given by:

$$I_{ij}(k) \propto S(k) \sum_{n=1}^{N} \sqrt{R_n R_R} \left( \cos 2k\, z_n \right) \qquad (1)$$

where $S(k)$ is the optical power spectral density of the swept light source 510 defined as a function of wavenumber $k$. $R_n$ is the reflectivity of the $n$-th retinal layer, and $R_R$ is the reflectivity of the reference mirror 335, and $z_n$ is a value representative of the optical path length difference between the optical path length of the reference arm 330 and the optical path length of the sample arm up to $n$-th retinal layer of the eye 30 when the eye 30 is being imaged. Fig. 6B illustrates an example of an interferogram 610 which is generated by a photodetector element 75 of the array as the wavelength of the light of the swept light source 510 is varied across its plurality of wavelength values.

Returning to Fig. 5, the FF-SS-OCT imaging system 500 may, as in the present example embodiment, further comprise a bandpass filter (not illustrated) arranged to receive the interferogram 610 generated by each photodetector element 75 of the array 70 and generate a filtered interferogram by extracting frequency components of the interferogram 610 within a passband of the bandpass filter. The passband of the bandpass filter may correspond to an imaging range taken along the propagation direction of the beams 90 in the eye 30, and the extracted frequency components are generated by the interference of light reflected from the region 20 of the eye 30 that is within the imaging range, with the reference light 322. In addition, the FF-SS-OCT imaging system 500 may further comprise a sampling module (not illustrated) that is arranged to receive the filtered interferogram of each photodetector element 75 and acquire samples of the filtered interferogram.

For a swept-source OCT imaging system, the depth reflection profile is determined by the inverse Fourier transform of the measured interferogram over wavenumber. Accordingly, the tomographic image data generating module 580 may, as in the FF-SS-OCT imaging system 500 of Fig. 5, be further arranged to perform an inverse Fourier transform on the samples of each filtered interferogram to generate an A-scan from the interferogram. As each photodetector element 75 may be mapped to a corresponding location in the region 20 of the eye 30, the A-scan obtained from an interferogram provides depth profile information for the location in the region 20 that corresponds the photodetector element 75 generating the interferogram. The tomographic image data generating module 580 is arranged to similarly obtain a plurality of A-scans corresponding to the plurality of respective photodetector elements 75 in the array 70. Furthermore, the tomographic image generating module 580 may, as in the FF-SS-OCT imaging system 500 of the present example embodiment, be arranged to combine the plurality of A-scans to form a B-scan or a C-scan of the region 20 of the eye 30.

Figure 6B also illustrates an example of an A-scan 620 that is obtained by processing the interferogram 610 which, by way of an example, is generated by the photodetector element 75 located at photodetector element location (1,1) in the array 70. After obtaining an A-scan 620 from the interferogram 610 generated by each photodetector element 75 of the array 70, the tomographic image data generating module 580 may combine the plurality of A-scans by arranging the plurality of A-scans based on the locations in the array 70 of the photodetector elements 75 from which the plurality of A-scans are derived. Figure 6B illustrates a three-dimensional tomographic image 630 (corresponding to a C-scan) of the region 20 that comprises A-scan 620.

It should be noted that confocal OCT imaging system 300 is not limited to being a full-field swept source OCT imaging system. In some example embodiments, the confocal OCT imaging system 300 may take the form of a time-domain OCT (TD-OCT) imaging system, which comprises a reference arm along which reference light generated by the beam splitter 320 propagates, and an optical path adjustment mechanism which is arranged to vary an optical path length of the reference arm (herein referred to as the reference arm length). In such a TD-OCT imaging system, which is described in more detail below, the illumination module 50 is arranged to perform a plurality of scans of the region 20 of the subject's eye 30, while the optical path length is being varied by the optical path adjustment mechanism, by scanning the plurality of non-overlapping beams 90 across the region 20 such that each point in the region 20 is illuminated by a respective one of the plurality of non-overlapping beams 90 during each of the scans. Furthermore, the illumination module may be arranged to perform a scan for each reference arm length of a plurality of predefined reference arm lengths.

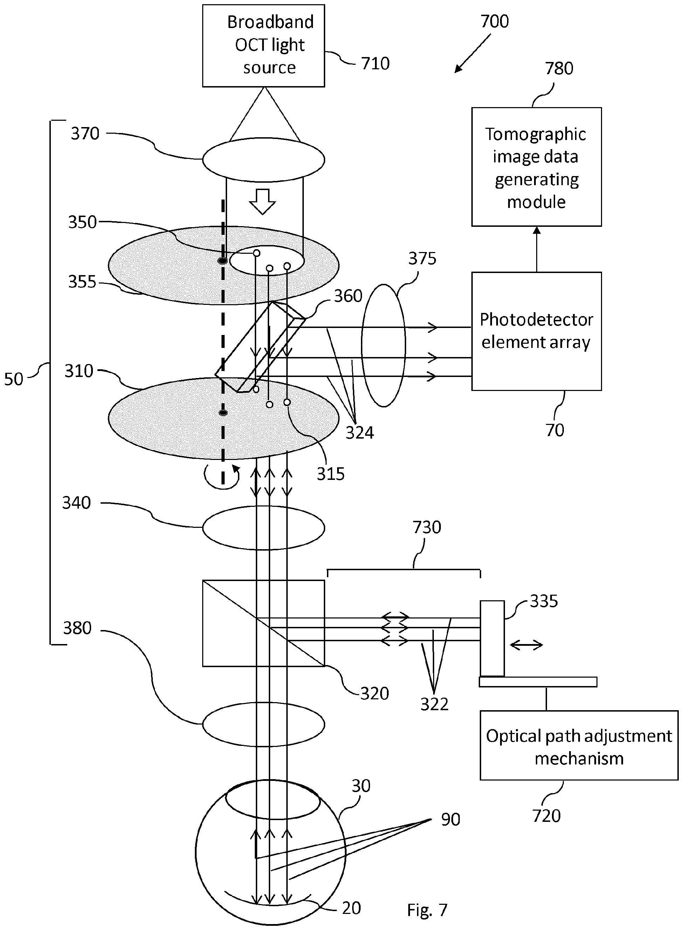

Figure 7 is a schematic illustration of an example implementation of the confocal OCT imaging system 300 of Fig. 3 as a full-field time-domain OCT (FF-TD-OCT) imaging system. The FF-TD-OCT imaging system 700 of Fig. 7 comprises an illumination module 50 that comprises the mask 310, the plurality of lenslets 350, the collimating lenses 370, 340 and the focusing lens 380, which are identical to the correspondingly labelled components in the background examples and example embodiments described above. Furthermore, FF-TD-OCT imaging system 700 comprises a beam splitter 320, a beam splitter 360, and a photodetector element array 70 that are the same as the correspondingly labelled components in the background examples and example embodiments described above. The description of these common components and the variants thereof provided above will not be repeated here. The FF-TD-OCT imaging system 700 further comprises a tomographic imaging data generating module 780, which is described in more detail below.

The FF-TD-OCT imaging system 700 of Fig. 7 is similar to the FF-SS-OCT imaging system 500 of Fig. 5 in many respects but differs from the example embodiment of Fig. 5 in that a broadband OCT light source 710, which is arranged to emit low-coherence light, is provided in place of the full-field swept light source 510 of Fig. 5. Furthermore, the FF-TD-OCT imaging system 700 comprises an optical path adjustment mechanism 720, which is arranged to vary an optical path length of a reference arm 730 through a plurality of predefined optical path lengths. The optical path adjustment mechanism 720 may, as shown in Fig. 7, comprise a translation stage which is arranged to vary the optical path length of the reference arm 730 by translating the reference mirror 335 through a plurality of predefined positions (corresponding to the plurality of predefined reference arm lengths) in order to vary the optical path distance between beam splitter 320 and the reference mirror 335. However, the optical path adjustment mechanism 720 is not limited in this regard, and may comprise any suitable mechanism for varying the reference arm lengths (namely, the optical path distance between beam splitter 320 and the reference mirror 335 in Fig. 7). The optical path adjustment mechanism 720 may, as in the present background example, vary the reference arm length in a step-wise manner but may alternatively vary the reference arm length in a continuous manner.

In Fig. 7, the illumination module 50 may, as in the present example, be arranged to perform a scan of the region 20 of the eye 30 for each reference arm length of the plurality of predefined reference arm lengths, as the path adjustment mechanism 720 varies the optical path length of the reference arm 730. Furthermore, for each scan performed at each of the plurality of predefined reference arm lengths, the illumination module 50 is arranged to scan the plurality of non-overlapping beams 90 across the region 20 such that each point in the region 20 is illuminated by a respective one of the non-overlapping beams 90. In this manner, the entire area of the region 20 can be illuminated by the plurality of non-overlapping beams 90 at least once for each predefined reference arm length.

In the FF-SS-OCT imaging system 500 of Fig. 5, the mask 310 is arranged to rotate at a rate that is based on the sweep rate of the swept light source 510 in order to ensure that the entire area of the region 20 of the eye 30 is scanned by the plurality of non-overlapping beams 90 for each wavelength of a plurality of wavelengths of the light output by the swept light source 510. However, in the FF-TD-OCT imaging system 700 of Fig. 7, the mask 310 may instead be arranged to rotate at a speed that is based on the speed at which the optical path adjustment mechanism 720 varies the optical path length of the reference arm 730 through the plurality of predefined reference arm lengths. The speed at which the optical path adjustment mechanism 720 varies the optical path length of the reference arm 730 through the plurality of predefined reference arm lengths determines the duration of time for which the reference arm length is set to each predefined reference arm length. For example, as the optical path adjustment mechanism 720 varies the optical path length of the reference arm 730 through each reference arm length of the plurality of predefined reference arm lengths, the mask 310 may, as in the present background example, be arranged to rotate at a rate such that, for each predefined reference arm length, each point in the region 20 is illuminated by a respective one of the plurality of non-overlapping beams 90. In other words, for each predefined reference arm length, the plurality of non-overlapping beams 90 is scanned across the entire area of the region 20. In this manner, the entire area of the region 20 is imaged at a plurality of different reference path lengths, thereby giving depth profile information for the region 20.

In the FF-SS-OCT imaging system 500 of Fig. 5, the photodetector element array 70 is arranged to measure interference light 324 corresponding to each wavelength of light output by the swept light source 510. In contrast, in the FF-TD-OCT imaging system 700 of Fig. 7, the photodetector elements 75 in the array 70 are instead arranged to measure the interference light 324 for each reference arm length of the plurality of predefined reference arm lengths of the FF-TD-OCT imaging system 700, as the optical adjustment mechanism varies the optical arm length of the reference arm 730. More specifically, each photodetector element 75 may, as in the present background example, have an integration time substantially equal to a duration of each scan and is arranged to make a respective single measurement of the interference light 324 during each scan. The measurement is therefore based on the interference light 324 received by the photodetector element 75 when the optical path length of the reference arm 730 is set to a reference arm length of the plurality of predefined reference arm lengths. However, as with FF-SS-OCT imaging system 500, it should be understood that the integration time of each photodetector element 75 need not be substantially equal to the duration of the scan. For example, in some examples, the integration time of each photodetector element 75 may be longer than the duration of the scan, such that multiple scans of the region 20 can be performed by the illumination module 50 for a single reference arm length before a measurement is acquired by a photodetector element 75.

Upon each photodetector element 75 obtaining a plurality of measurements of the interference light 324 corresponding to the plurality of predefined reference arm lengths, the tomographic image data generating module 780 may, as in the present background example, be further arranged to generate tomographic image data based on the plurality of measurements. In particular, each measurement of the interference light 324 corresponds to reflectivity information for a particular depth of the eye 30, wherein the depth depends on the reference arm length that is used to generate the interference light 324. Accordingly, the tomographic image data generating module 780 may, as in the present background example, generate an A-scan for each photodetector element 75 based on the plurality of measurements made by the photodetector element 75 using the plurality of respective reference arm lengths. The tomographic image data generating module 780 may further array the plurality of A-scans to form a B-scan or a C-scan.

The photodetector element array 70 used in the FF-TD-OCT imaging system 700 of Fig. 7 may be identical to the array described in relation to Fig. 5. However, unlike the FF-SS-OCT imaging system 500 (where each photodetector element 75 of the array generates an interferogram), for the FF-TD-OCT imaging system 700 of Fig. 7, as a plurality scans are performed over the plurality of respective predefined reference arm lengths, each photodetector element 75 of the array 70 generates an electrical signal that represents the reflectivity information of the eye 30 over different depths within the eye 30. The tomographic image data generating module 780 may further generate, based on the electrical signal, an A-scan that provides the depth profile for the point on the region 20 that corresponds to the photodetector element 75 detecting the electrical signal.

As with the FF-SS-OCT imaging system 500 of Fig. 5, it should be noted that each photodetector element 75 is not limited to making a single measurement of the interference light 324 for each reference arm length. In particular, as the optical length of the reference arm is set to each reference arm length of the plurality of predefined reference arm lengths, the illumination module 50 may be arranged to perform a plurality of scans for each reference arm length, wherein for each scan, the entire area of the region 20 of the eye 30 is illuminated by the plurality of non-overlapping beams 90 as the mask 310 is rotated. Furthermore, each photodetector element 75 of the array 70 may be arranged to generate, for each reference arm length, a plurality of measurements of the interference light 324 corresponding to the plurality of respective scans performed by the illumination module 50 while the optical length of the reference arm 730 is set to the reference arm length. Furthermore, the tomographic image data generating module 780 may average the plurality of measurements made for each reference arm length to calculate an averaged measurement for each reference arm length. The tomographic image data generating module 780 may further be arranged to generate the tomographic data using the averaged measurement calculated for each photodetector element 75 for each reference arm length.

As with the FF-SS-OCT imaging system 500, the integration time need not be substantially equal to the duration of the scan. For example, in an alternative implementation of the FF-TD-OCT imaging system 700, the photodetector elements 75 may have an integration time which is smaller than a duration of the scan, and may be arranged to make concurrent measurements of the interference light 324 in each of a plurality of integration time intervals during the scan. Furthermore, the tomographic image data generating module 780 may in that case be arranged to combine the measurements made by the photodetector elements 75 in each of the integration time intervals in order to generate a combined measurement for the scan. In the FF-TD-OCT imaging system 700 of the present background example, each scan is performed at a predetermined reference arm length, and the combined measurement for the scan therefore provides reflectivity information of the region 20 at a particular depth within the region 20. In addition, the tomographic image data generating module 780 may, as in the present background example, further generate, based on the combined measurements made by the photodetector elements 75 in each of the integration time intervals, respective partial tomographic image data defining a tomographic image of a respective portion of the region 20. The partial tomographic image data may correspond to a cross-section or "slice" of the region 20 at a particular depth within the eye 30 (the depth being dependent on the reference arm length that is used when the scan is performed). The tomographic image data generating module 780 may, as in the present background example, be further arranged to combine the partial tomographic image data obtained using different reference lengths in order to generate the tomographic image data defining the tomographic image of the region 20 of the subject's eye 30.

In some example embodiments, the confocal OCT imaging system 300 of Fig. 3 may take the form of a line-field OCT imaging system. In these example embodiments, the light source 40 of Fig. 3 takes the form of a line-field OCT light source, which is arranged to provide a line of light to the illumination module, and the illumination module is arranged to perform a scan of the region 20 of the subject's eye 30 by scanning the plurality of non-overlapping beams 90 across each of a plurality of lines crossing the region 20 such that each point in the region 20 is illuminated by a respective one of the plurality of non-overlapping beams 90 during the scan.

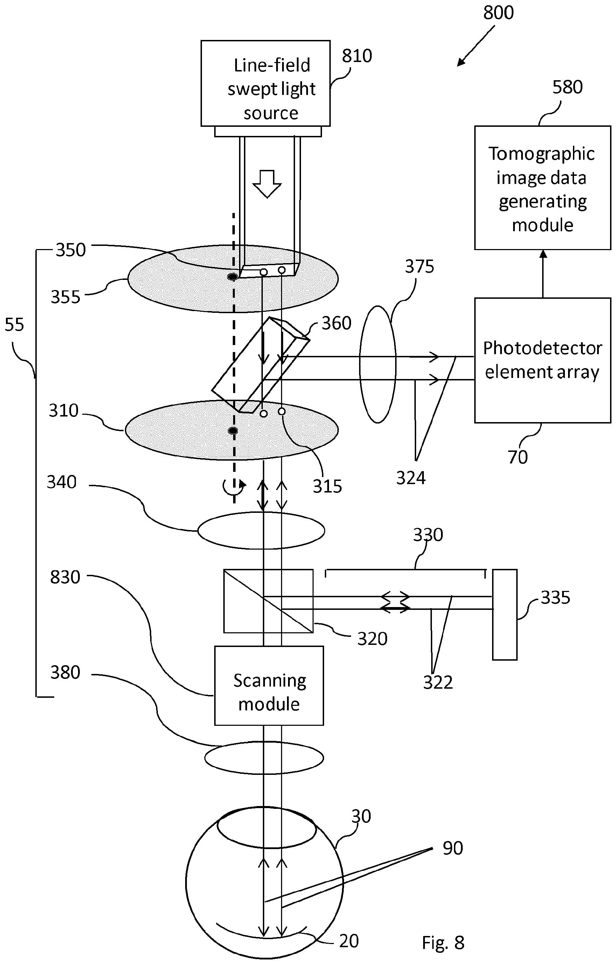

Figure 8 is a schematic illustration of an example implementation of the confocal OCT imaging system 300 of Fig. 3 as a line-field swept-source OCT (LF-SS-OCT) imaging system. The LF-SS-OCT imaging system 800 in Fig. 8 comprises an illumination module 55 that comprises a mask 310, a lenslet disk 355 comprising a plurality of lenslets 350, collimating element 340, and a focusing lens 380 that are identical to the corresponding labelled components in the example embodiments described above. In addition, the LF-SS-OCT imaging system 800 may further comprise, as shown Fig. 8, beam splitters 320, 360, a photodetector element array 70, tomographic image data generating module 580, and reference mirror 335 which are all identical to the correspondingly labelled components in FF-SS-OCT imaging system 500 of Fig. 5.

The LF-SS-OCT imaging system 800 further comprises a line-field swept light source 810, which, like the full-field swept light source 510 of the FF-SS-OCT imaging system 500, is arranged to generate light of varying wavelength. Furthermore, the illumination module 55 is arranged to perform a plurality of scans of the region 20 of the subject's eye 30, while the wavelength of the light is being varied, by scanning the plurality of non-overlapping beams 90 across the region 20 such that each point in the region 20 is illuminated by a respective one of the plurality of non-overlapping beams 90 during each of the scans. The photodetector element array 70 is further arranged to detect the interference light 324 generated during each of the scans. As with the FF-SS-OCT imaging system 500 described above, the illumination module 55 of the LF-SS-OCT imaging system 800 is arranged to perform a scan of the region 20 using light of each wavelength of a plurality of wavelengths output by the line-field swept light source 810.

The line-field swept light source 810, however, differs from the full-field swept light source 510 in that, instead of providing a full-field two-dimensional illumination, the line-field swept light source 810 is arranged to project a line of the light, which would simultaneously illuminate all points along a line of a surface onto which the line of light is projected.

In the example embodiment of Fig. 8, the line-field swept light source 810 projects a line of light onto the plurality of lenslets 350 that focus light incident thereon onto a plurality of respective transmissive portions 315 of the mask 310, and the light propagating through the plurality of transmissive portions 315 form the plurality of non-overlapping beams 90. However, in some example embodiments, the LF-SS-OCT imaging system 800 does not comprise the plurality of lenslets 350 and the line-field swept source 810 instead projects the line of light directly onto the mask 310 to form the plurality of non-overlapping beams 90. The line-field OCT light source 810 may, as in the present example embodiment, comprise a swept light source, a collimating lens and cylindrical lens (not shown) that are arranged to generate the line of light.

As with the FF-SS-OCT imaging system 500 described above with reference to Fig. 5, the mask 310 may, as in the example embodiment of Fig. 8, be arranged to rotate at an angular speed that is dependent on the rate at which the wavelength of the light of the line-field swept light source 810 is varied across the plurality of wavelengths. In addition, the mask 310 is arranged to allow the light from the line-field swept light source 810 to propagate through the transmissive portions 350 of the mask 310 to form the plurality of non-overlapping beams 90.

The LF-SS-OCT imaging system 800 further differs from the FF-SS-OCT imaging system 500 in that the illumination module 55 of the LF-SS-OCT imaging system 800 additionally comprises a scanning module 830 that is arranged to receive the plurality of non-overlapping beams 90 propagating from the transmissive portions 315 of mask 310 and scan the plurality of non-overlapping beams 90 across the region 20 of the eye 30. The scanning module 830 may, as in the present example embodiment, be arranged to scan the plurality of non-overlapping beams in a single direction across the region 20 of the eye 30. The scanning module 830 may, as in the present example embodiment, comprise a galvanometer mirror (not shown) that is arranged to scan the plurality of non-overlapping beams 90 in the aforementioned direction by varying an inclination angle of the galvanometer mirror. The galvanometer mirror may be rotated by an actuation mechanism (not shown), such as a motor, so as to vary the optical path of the plurality of non-overlapping beams 90 and therefore vary the scan location within the region 20 of the eye 30 during imaging. In addition, the scan angle of the light beams scanned into the eye 30 may, as in the present example embodiment, depend on the inclination angle of the galvanometer mirror, wherein the inclination angle indicates the degree of rotation of the galvanometer mirror about its axis of rotation. Although the scanning module 830 is described to be a galvanometer mirror, it may alternatively comprise any other suitable scanning element, such as a micro-electromechanical system (MEMS) mirror or the like.

Due to the line illumination provided by the line-field swept light source 810, the plurality of non-overlapping beams 90 which propagate from the mask 310 may, as in the example embodiment of Fig. 8, illuminate each point of a line on the surface of the galvanometer mirror. By varying the inclination angle of the galvanometer mirror, the plurality of non-overlapping beams 90 received from the mask 310 can be scanned by the galvanometer mirror across the region 20 of the eye 30 via the focusing 380 lens. More specifically, for each inclination angle of the galvanometer mirror, each point of a line extending across the region 20 of the eye 30 is illuminated by a respective one of the plurality of non-overlapping beams 90 due to the rotation of the mask 310. By varying the inclination angle of the galvanometer mirror as the mask 310 is rotated, the plurality of non-overlapping beams 90 from the mask 310 can be scanned by the galvanometer mirror across the region 20 of the eye 30 in a direction orthogonal to the line extending the region 20 that is illuminated due to the rotation of the mask 310.

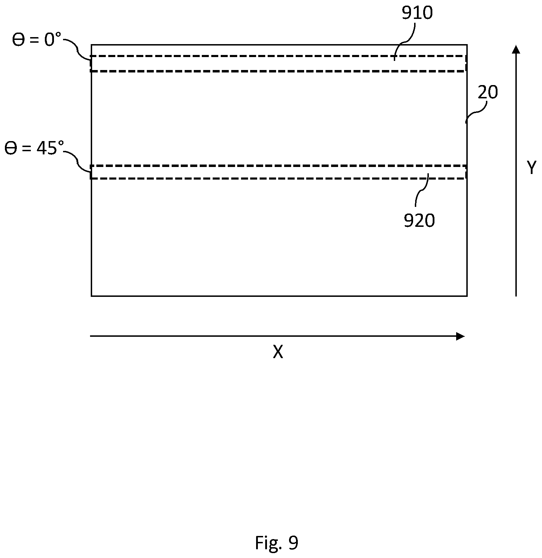

Figure 9 is an example illustration of a pattern by which the plurality of the non-overlapping beams 90 can be scanned across the region 20 of the eye 30 in the LF-SS-OCT imaging system 800. Line 910 in Fig. 9 illustrates a line of the region 20 that is scanned by the illumination module 55 when the inclination angle, $\theta$, of the galvanometer mirror is set to 0°. Each point on the line 910 is scanned by a respective one of the plurality of non-overlapping beams 90 as a result of the rotation of the mask 310. Line 920 in Fig. 9 illustrates a line in the region 20 that is scanned by the illumination module 55 when the inclination angle of the galvanometer mirror is set to 45°. As shown in Fig. 9, the rotation of the mask 310 causes the plurality of non-overlapping beams 90 to be scanned in a horizontal direction (namely, along the X-axis), while the adjustment of the inclination angle of the galvanometer mirror causes the plurality of non-overlapping beams 90 to be scanned in the vertical direction (namely, along the Y-axis). Accordingly, by combining the rotation of the mask 310 with the simultaneous variation of the inclination angle $\theta$ of the galvanometer mirror, the illumination module is able to perform a scan of the region 20 of the eye 30 by scanning the plurality of non-overlapping beams 90 across each of a plurality of lines extending across the region 20 such that each point in the region 20 is illuminated by a respective one of the plurality of non-overlapping beams 90 during the scan.

For a mask 310 having a given pattern of transmissive portions, a predetermined number of revolutions of the mask 310 is required to ensure that each point of a line extending across the region 20 is illuminated by the plurality of non-overlapping beams 90. Furthermore, rotating the mask 310 at a higher rate allows every point of the line to be illuminated within a shorter period of time. Accordingly, for a given sweep rate of the swept light source 810, the rate at which the inclination angle $\theta$ of the galvanometer mirror is varied during the scan and the rotational speed of the mask 310 may be selected so as to ensure that a scan of the region 20 is performed using each wavelength of the plurality of wavelengths as the line-field swept light source 810 varies the wavelength of its output light. More generally, it should be understood that for the LF-SS-OCT imaging system 800, the rotation speed of the mask 310, the scanning speed of the scanning module 830, and the sweep rate of the line-field swept light source 810 can be set in a combination which ensures that a scan of the entire region 20 is performed using light of each wavelength of the plurality of wavelengths of the line-field swept light source 810.

For the LF-SS-OCT imaging system 800 of Fig. 8, the photodetector element array 70 and the tomographic image data generating module 580 may function in the same manner as previously described for the FF-SS-OCT imaging system 500. It is noted that Fig. 8 illustrates merely one example implementation of the LF-SS-OCT imaging system, which is based on same arrangement shown in Fig. 3. However, a LF-SS-OCT imaging system according to an example embodiment may alternatively be implemented based on the arrangement shown in Fig. 4, or using any of the variations described in relation to Figs. 3 and 4. Furthermore, in some example embodiments, instead using a scanning module 830 to scan the beams propagating from the mask 310 across the region 20 of the eye 30, the scanning module 830 may alternatively be arranged to receive the line of light (i.e. line illumination) from the line-field swept light source 810 and scan the line of light across the rotating mask 310. Light from the light source 810 which passes through the transmissive portions 315 of the mask 310 may form the plurality of non-overlapping beams 90 that is directed onto the region 20 of the eye 30 during the scan.

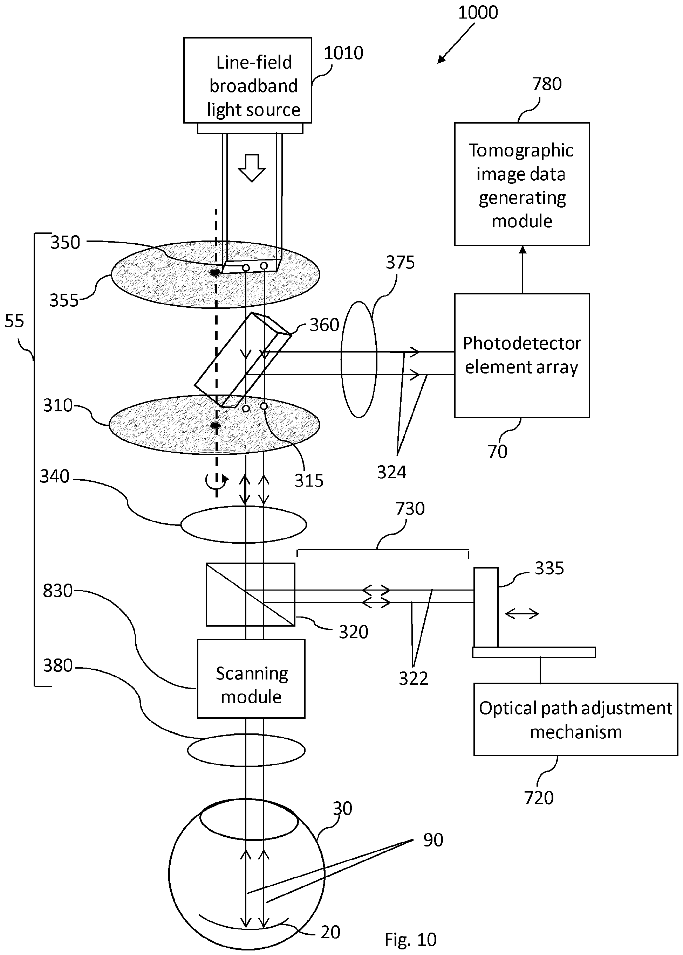

In some background examples, the confocal OCT imaging system 300 of Fig. 3 may take the form of a line-field time-domain (LF-TD) OCT imaging system. Figure 10 is a schematic illustration of an example implementation of the confocal OCT imaging system 300 of Fig. 3 as a LF-TD OCT imaging system. The LF-TD OCT imaging system 1000 illustrated in Fig. 10 has an illumination module 55 which comprises a mask 310, a lenslet disk 355 comprising a plurality of lenslets 350, a scanning module 830, a collimating lens 340, and a focusing lens 380 which are identical to the correspondingly labelled components of the LF-SS-OCT imaging system 800 in Fig. 8. In addition, the LF-TD OCT imaging system 1000 in Fig. 10 also comprises beam splitters 320, 360 and a photodetector element array 70 which are also identical to the correspondingly labelled components in previous background examples and embodiments. Furthermore, the LF-TD OCT imaging system 1000 in Fig. 10 comprises a tomographic image data generating module 780 that is identical to the correspondingly labelled component in the FF-TD-OCT imaging system 700 of Fig. 7. In particular, the tomographic image data generating module 780 for time domain OCT systems may perform identical or similar processing operations on the measurements obtained by the array of photodetector elements 70 to obtain the tomographic image data.

The LF-TD OCT imaging system 1000 is similar to the LF-SS-OCT imaging system 800 of Fig. 8 in many respects but differs from the example embodiment of Fig. 8 by the configuration of the light source and the arrangement of the reference arm. More specifically, the LF-TD OCT imaging system 1000 comprises, in place of a line-field swept light source, a line-field broadband OCT light source 1010, which is arranged to project a line of low coherence light. In the example of Fig. 10, the line-field broadband OCT light source 1010 projects a line of light onto the plurality of lenslets 350 which focus the light onto a plurality of respective transmissive portions 315 of the mask 310, and the light propagating through the plurality of transmissive portions 315 forms the plurality of non-overlapping beams 90. However, the LF-TD OCT imaging system 1000 need not comprise the plurality of lenslets 350, in which case the line-field broadband OCT light source 1010 may project the line of light generated thereby directly onto the mask 310 to form the plurality of non-overlapping beams 90. The line-field broadband OCT light source 1010 may, as in the present background example, comprise a broadband light source, a collimating lens and cylindrical lens (not shown) in order to create the line illumination.

Furthermore, LF-TD OCT imaging system 1000 comprises a reference arm 730 having a reference mirror 335 and an optical path adjustment mechanism 720 which are identical to the correspondingly labelled components of the FF-TD-OCT imaging system 700 described above with reference to Fig. 7. In addition, as with the LF-TD OCT imaging system 700, the illumination module 55 of the LF-TD OCT imaging system 1000 is also arranged to perform a plurality of scans of the region 20 of the subject's eye 30 while the optical path length of the reference arm 730 is being varied by the optical path adjustment mechanism 720, by scanning the plurality of non-overlapping beams 90 across the region 20 such that each point in the region 20 is illuminated by a respective one of the plurality of non-overlapping beams 90 during each of the scans. As with the FF-TD-OCT imaging system 700, for each reference arm length of a plurality of predefined reference arm lengths, at least one scan of the region 20 is performed in the present background example by the illumination module 55.

The LF-TD OCT imaging system 1000 of Fig. 10 is also similar to the LF-SS-OCT imaging system 800 of Fig. 8, in that the illumination module 55 of the LF-TD OCT imaging system 1000 is arranged to scan the plurality of non-overlapping beams 90 of light across the entire area of the region 20 in the same manner as the LF-SS-OCT imaging system 800. In particular, LF-TD OCT imaging system 1000 also combines the rotation of the mask 310 with the single direction scanning performed by a scanning module 830 (which is the same as the similarly numbered component of the LF-SS-OCT imaging system 800 ) in order to scan the plurality of non-overlapping beams 900 across each of a plurality of lines extending across the region 20 such that each point in the region 20 is illuminated by a respective one of the plurality of non-overlapping beams 90 during the scan. The illumination module 55 of the LF-TD OCT imaging system 1000 differs from that of the LF-SS-OCT imaging system 800 in that, instead of performing a scan for each wavelength of the plurality of wavelengths of light output by the line-field swept light source 810, the illumination module 55 of the LF-TD OCT imaging system 1000 performs a scan for each reference arm length of a plurality of predefined reference arm lengths as the optical path length of the reference arm 730 is varied by the optical path adjustment mechanism 720.

It is noted that the confocal OCT imaging system illustrated of Fig. 10 is merely an example implementation of the LF-TD-OCT imaging system that is based on the arrangement shown in Fig. 3. However, LF-TD-OCT imaging system 1000 may alternatively be implemented based on the arrangement shown in Fig. 4, or any other alternative arrangement previously described.

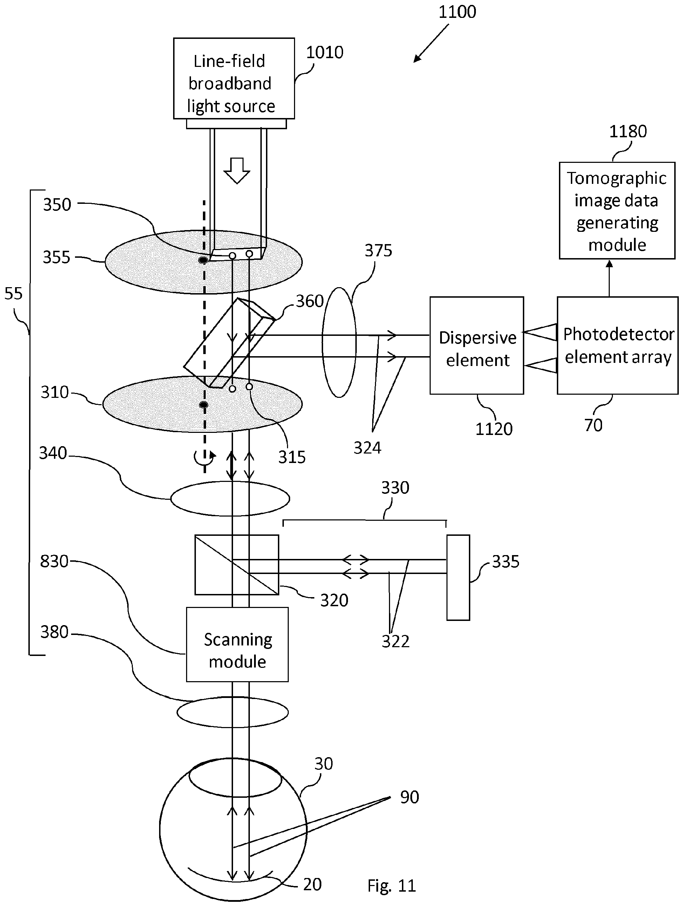

In some background examples, the confocal OCT imaging system 300 of Fig. 3 may take the alternative form of a line-field spectral-domain OCT (LF-SD-OCT) imaging system. Figure 11 is a schematic illustration of an example implementation of the confocal OCT imaging system 300 of Fig. 3 as a LF-SD-OCT imaging system. As shown in Fig. 11, the LF-SD-OCT imaging system 1100 comprises a line-field broadband light source 1010 that is arranged to project a line of the low coherence light onto the plurality of lenslets 350 in the same manner as described above in relation to the LF-TD-OCT imaging system 1000 of Fig. 10. However, as with the example embodiments and background examples described above, the LF-SD-OCT imaging system 1100 need not comprise the plurality of lenslets 350, and the line-field light broadband source 1010 may directly project the line of light onto the mask 310.

The LF-SD-OCT imaging system 1100 comprises an illumination module 55 having a mask 310, a lenslet disk 355 comprising a plurality of lenslets 350 and a scanning module 830 that are identical to the corresponding labelled components in the LF-SS-OCT imaging system 800 of Fig. 8. In addition, the LF-SD-OCT imaging system 1100 may, as shown in Fig. 11, further comprise beam splitters 320, 360, a photodetector element array 70, a collimating lens 340, a reference arm 330 (comprising a reference mirror 335) and a focusing lens 380, which are all identical to the correspondingly labelled components as previously described.

The LF-SD-OCT imaging system 1100 of Fig. 11 further comprises a dispersive element 1120, which is arranged to spatially separate frequency components of the interference light 324. Furthermore, the photodetector element array 70 is arranged to detect respective frequency components of the interference light 324. The LF-SD-OCT imaging system 1100 may, as in the present background example, further comprise a slit aperture (not shown), which is placed in a focal plane of the LF-SD-OCT imaging system 1100, and arranged to receive the interference light 324 from the beam splitter 360 and spatially filter the interference light 324 by suppressing out-of-focus light. As a result of the spatial filtering performed by the slit aperture, the interference light 324 may be projected along a line onto the dispersive element 1120. The dispersive element 1120 may, as in the present background example, comprise a diffraction grating that is arranged to receive the interference light 324 passed by the slit aperture and disperse the interference light 324 into its frequency components onto the photodetector element array 70. The frequency components of the interference light 324 may, as in the present background example, be projected onto the photodetector element array 70 in a direction orthogonal to the line of interference light 324 received by the diffraction grating.

In the LF-SD-OCT imaging system 1100 of Fig. 11, the photodetector element array 70 is further arranged to measure the dispersed interference light and, in particular, detect the frequency components of the interference light 324. The photodetector element array 70 may, as in the present background example, be further be arranged generate a spectral interferogram for each point of the region 20 as the illumination module 55 scans the plurality of non-overlapping beams 90 across each of a plurality of lines crossing the region 20 such that each point in the region 20 is illuminated by a respective one of the plurality of non-overlapping beams 90 during the scan.

The LF-SD-OCT imaging system 1100 further comprises a tomographic image data generating module 1180 that is arranged to generate the tomographic image data by calculating an inverse Fourier transform of the detected frequency components of the interference light 324 that have been spatially separated by the dispersive element 1020. For example, the tomographic image data generating module 1180 may, as in the present background example, perform Fourier transform on the interferogram that is detected by the photodetector element array 70 for each point of region 20 during the scan. The Fourier transform of the interferogram for a point of the region provides the A-scan profile for that point. Upon obtaining an A-scan for each point of the region 20, the tomographic image data generating module 1180 may further combine the A-scans form a B-scan or a C-scan of the region 20.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Software embodiments of the examples presented herein may be provided as, a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, floppy diskettes, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some embodiments may also be implemented by the preparation of application-specific integrated circuits, field-programmable gate arrays, or by interconnecting an appropriate network of conventional component circuits.

Some embodiments include a computer program product. The computer program product may be a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above described example embodiments, but should be defined only in accordance with the following claims and their equivalents.

Further, the purpose of the Abstract is to enable the Patent Office and the public generally, and especially the scientists, engineers and practitioners in the art who are not familiar with patent or legal terms or phraseology, to determine quickly from a cursory inspection the nature and essence of the technical disclosure of the application. The Abstract is not intended to be limiting as to the scope of the example embodiments presented herein in any way. It is also to be understood that any procedures recited in the claims need not be performed in the order presented.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

The apparatuses described herein may be embodied in other specific forms without departing from the characteristics thereof. The foregoing embodiments are illustrative rather than limiting of the described systems and methods. Scope of the apparatuses described herein is thus indicated by the appended claims, rather than the foregoing description.

**Claims**

1. A confocal optical coherence tomography, OCT, imaging system (10, 300, 400, 500, 800) for imaging a region (20) of a subject's eye (30), the confocal OCT imaging system (10, 300, 400, 500, 800) comprising:

   a light source (510, 810) arranged to generate light;
   an illumination module (50, 55) comprising a mask (310) having a plurality of transmissive portions (315) through which the light from the light source (510, 810) can propagate to form respective non-overlapping beams (90) of light when the mask (310) is illuminated by the light source (510, 810), the illumination module (50, 55) being arranged to perform a scan of the region (20) of the subject's eye (30) by scanning the plurality of non-overlapping beams (90) across the region (20) such that each point in the region (20) is illuminated by a respective one of the plurality of non-overlapping beams (90) during the scan;
   an interferometer (60) comprising a beam splitter (320, 410) arranged to generate reference light (322, 414) based on the light generated by the light source (510, 810), the interferometer (60) being arranged to generate interference light (324, 445) by combining, for each beam, light from the beam which has been reflected from the region (20) with the reference light (322, 414) generated by the beam splitter (320, 410);
   a photodetector element array (70) arranged to detect the interference light (324, 445) generated during the scan; and
   a tomographic image data generating module (80, 580) arranged to generate tomographic image data (620, 630) defining a tomographic image of the region (20) based on the detected interference light (324, 445), wherein the ophthalmic OCT imaging system is a swept-source OCT imaging system (10, 300, 400, 500, 800),
   the light source is a swept light source (510, 810) arranged to generate light of varying wavelength,
   the illumination module (50, 55) is arranged to perform a plurality of scans of the region (20) of the subject's eye (30), while the wavelength of the light is being varied, by scanning the plurality of non-overlapping beams (90) across the region (20) such that each point in the region (20) is illuminated by a respective one of the plurality of non-overlapping beams (90) during each of the scans,
   the photodetector elements (70) are arranged to detect the interference light (324) generated during each of the scans,
   **characterised in that**,
   as the wavelength of the light output by the swept light source (510, 810) is swept across a plurality of wavelength values, the illumination module (50, 55) is arranged to perform the plurality of scans of the region (20) by performing a plurality of scans for each wavelength of light, wherein, for each scan, the entire area of the region (20) is illuminated by the plurality of non-overlapping beams (90) of the wavelength as the mask (310) is rotated, and **in that** each photodetector element of the array (70) is arranged to detect the interference light (324) generated during each of the scans by generating, for each wavelength, a plurality of measurements of the interference light (324) corresponding to the plurality of scans performed using light of that wavelength, and **in that** the tomographic image data generating module (80, 580) is further arranged to perform, for each photodetector element and for each wavelength, a determination of an averaged measurement by averaging the plurality of measurements obtained by the photodetector element for the wavelength, and generate the tomographic image data based on the averaged measurement for each photodetector and for each wavelength.

2. A confocal optical coherence tomography, OCT, imaging system (10, 300, 400, 500, 800) for imaging a region (20) of a subject's eye (30), the confocal OCT imaging system (10, 300, 400, 500, 800) comprising:

   a light source (510, 810) arranged to generate light;
   an illumination module (50, 55) comprising a mask (310) having a plurality of transmissive portions (315) through which the light from the light source (510, 810) can propagate to form respective non-overlapping beams (90) of light when the mask (310) is illuminated by the light source (510, 810), the illumination module (50, 55) being arranged to perform a scan of the region (20) of the subject's eye (30) by scanning the plurality of non-overlapping beams (90) across the region (20) such that each point in the region (20) is illuminated by a respective one of the plurality of non-overlapping beams (90) during the scan;
   an interferometer (60) comprising a beam splitter (320, 410) arranged to generate reference light (322, 414) based on the light generated by the light source (40, 510, 710, 810, 1010), the interferometer (60) being arranged to generate interference light (324, 445) by combining, for each beam, light from the beam which has been reflected from the region (20) with the reference light (322, 414) generated by the beam splitter (320, 410);

a photodetector element array (70) arranged to detect the interference light (324, 445) generated during the scan; and

a tomographic image data generating module (80, 580) arranged to generate tomographic image data (620, 630) defining a tomographic image of the region (20) based on the detected interference light (324, 445), wherein the ophthalmic OCT imaging system is a swept-source OCT imaging system (10, 300, 400, 500, 800),

the light source is a swept light source (510, 810) arranged to generate light of varying wavelength,

the illumination module (50, 55) is arranged to perform a plurality of scans of the region (20) of the subject's eye (30), while the wavelength of the light is being varied, by scanning the plurality of non-overlapping beams (90) across the region (20) such that each point in the region (20) is illuminated by a respective one of the plurality of non-overlapping beams (90) during each of the scans,

the photodetector elements (70) are arranged to detect the interference light (324) generated during each of the scans,

**characterised in that**

the integration time of each photodetector element (70) is longer than the duration of each scan, such that multiple scans of the region (20) is performed using light of the same wavelength before a measurement is acquired by the photodetector element (70).

3. The confocal OCT imaging system (10, 300, 500, 800) according to claim 1 or claim 2, wherein the interferometer (60) has a reference arm (330) comprising a reference mirror (335), and the beam splitter (320) is arranged to extract light from each of the beams (90) and direct the extracted light to the reference mirror (335), wherein the mirror (335) is arranged to reflect the extracted light back to the beam splitter (320) as the reference light (322), and wherein the beam splitter (320) is arranged to generate the interference light (324) by combining, for each beam, light from the beam which has been reflected from the region (20) with the light reflected back to the beam splitter (320) by the mirror (335).

4. The confocal OCT imaging system (10, 400) according to claim 1 or claim 2, wherein the interferometer comprises a second beam splitter (420) and a reference arm (430) along which the reference light (414) is arranged to propagate from the beam splitter (410) to the second beam splitter (420), the second beam splitter (420) being arranged to generate the interference light (445) by combining, for each beam, light from the beam which has been reflected from the region (20) with the reference light (414) propagating along the reference arm (430).

5. The confocal OCT imaging system (10, 300, 400, 500, 800) according to any preceding claim, wherein the mask (310) is further arranged to allow the light which has been reflected from the region (20) of the subject's eye (30) to propagate to the photodetector element array (70) only through the transmissive portions (315) of the mask (310).

6. The confocal OCT imaging system (10, 300, 400, 500, 800) according to any preceding claim, wherein the light source is a full-field OCT light source (40, 510) arranged to provide full-field illumination to the illumination module (50, 55).

7. The confocal OCT imaging system according to any one of claims 1 to 5, wherein the light source is a line-field OCT light source (810) arranged to provide a line of light to the illumination module (50, 55), and wherein the illumination module (50, 55) is arranged to perform a scan of the region (20) of the subject's eye (30) by scanning the plurality of non-overlapping beams (90) across each of a plurality of lines (910, 920) crossing the region (20) such that each point in the region (20) is illuminated by a respective one of the plurality of non-overlapping beams (90) during the scan.

8. The confocal OCT imaging system (10, 300, 400, 500, 800) according to any preceding claim, wherein the mask (310) is arranged to rotate during the scan, and the transmissive portions (315) are distributed across the mask (310) such that each point in the region (20) is illuminated by a respective one of the plurality of non-overlapping beams (90) as the mask (310) rotates during the scan.

9. The confocal OCT imaging system (10, 300, 400, 500, 800) according to claim 8, wherein the plurality of transmissive portions (315) are distributed across the mask (310) in an Archimedean spiral.

10. The confocal OCT imaging system (10, 300, 400, 500, 800) according to claim 8 or claim 9, wherein the illumination module (50, 55) further comprises a plurality of lenslets (350), each of the lenslets (350) being arranged to direct light from the light source (40) through a respective one of the transmissive portions in the mask (310), wherein the plurality of lenslets (350) are arranged to rotate together with the mask (310) so as to maintain an alignment of each lenslet (350) with the respective transmissive portion (315) during rotation of the mask (310) and the plurality of lenslets (350).

11. The confocal OCT imaging system (10, 300, 400, 500, 800) according to any of claims 8 to 10, wherein the transmissive portions (315) of the mask (310) comprise at least one of pinholes or slits in the mask (310).

**Patentansprüche**

1. Konfokales Optische-Kohärenz-Tomographie-, OCT-, Abbildungssystem (10, 300, 400, 500, 800) zum Abbilden einer Region (20) eines Auges (30) einer Zielperson, wobei das konfokale OCT-Abbildungssystem (10, 300, 400, 500, 800) umfasst:

eine Lichtquelle (510, 810), die angeordnet ist zum Erzeugen von Licht;
ein Beleuchtungsmodul (50, 55), umfassend eine Maske (310) mit einer Vielzahl von durchlässigen Abschnitten (315), durch die sich das Licht von der Lichtquelle (510, 810) ausbreiten kann, um jeweilige nicht überlappende Strahlen (90) aus Licht zu bilden, wenn die Maske (310) von der Lichtquelle (510, 810) beleuchtet wird, wobei das Beleuchtungsmodul (50, 55) angeordnet ist zum Durchführen eines Abtastens der Region (20) des Auges (30) der Zielperson durch Abtasten der Vielzahl von nicht überlappenden Strahlen (90) über die Region (20), so dass jeder Punkt in der Region (20) während des Abtastens von einem entsprechenden der Vielzahl von nicht überlappenden Strahlen (90) beleuchtet wird;
ein Interferometer (60), umfassend einen Strahlteiler (320, 410), der angeordnet ist zum Erzeugen von Referenzlicht (322, 414) auf Grundlage des von der Lichtquelle (510, 810) erzeugten Lichts, wobei das Interferometer (60) angeordnet ist zum Erzeugen von Interferenzlicht (324, 445) durch Kombinieren, für jeden Strahl, von Licht von dem Strahl, der von der Region (20) reflektiert wurde, mit dem von dem Strahlteiler (320, 410) erzeugten Referenzlicht (322, 414);
eine Fotodetektorelement-Anordnung (70), die angeordnet ist zum Detektieren des Interferenzlichts (324, 445), das während des Abtastens erzeugt wird; und
ein Tomographiebilddaten-Erzeugungsmodul (80, 580), das angeordnet ist zum Erzeugen von Tomographiebilddaten (620, 630), die ein Tomographiebild der Region (20) auf Grundlage des detektierten Interferenzlichts (324, 445) definieren, wobei
das ophthalmische OCT-Abbildungssystem ein Schwenklichtquellen-OCT-Abbildungssystem (10, 300, 400, 500, 800) ist,
die Lichtquelle eine Schwenklichtquelle (510, 810) ist, die angeordnet ist zum Erzeugen von Licht mit variierender Wellenlänge,
das Beleuchtungsmodul (50, 55) angeordnet ist zum Durchführen einer Vielzahl von Abtastungen der Region (20) des Auges (30) der Zielperson, während die Wellenlänge des Lichts variiert wird, durch Abtasten der Vielzahl von sich nicht überlappenden Strahlen (90) über die Region (20), so dass jeder Punkt in der Region (20) durch einen entsprechenden Strahl der Vielzahl von sich nicht überlappenden Strahlen (90) während jeder der Abtastungen beleuchtet wird,
die Fotodetektorelemente (70) angeordnet sind zur Detektion des Interferenzlichtes (324), das während jeder der Abtastungen erzeugt wird,
**dadurch gekennzeichnet, dass**,
während die Wellenlänge des von der Schwenklichtquelle (510, 810) ausgegebenen Lichts über eine Vielzahl von Wellenlängenwerten abgetastet wird, das Beleuchtungsmodul (50, 55) angeordnet ist zum Durchführen der Vielzahl von Abtastungen der Region (20) durch Durchführen einer Vielzahl von Abtastungen für jede Wellenlänge des Lichts, wobei für jede Abtastung die gesamte Fläche der Region (20) durch die Vielzahl von nicht überlappenden Strahlen (90) der Wellenlänge beleuchtet wird, während die Maske (310) gedreht wird, und
dadurch, dass jedes Fotodetektorelement der Anordnung (70) angeordnet ist zum Detektieren des Interferenzlichts (324), das während jeder der Abtastungen erzeugt wird, durch Erzeugen, für jede Wellenlänge, einer Vielzahl von Messungen des Interferenzlichts (324) entsprechend der Vielzahl von Abtastungen, die unter Verwendung von Licht dieser Wellenlänge durchgeführt werden, und
dadurch, dass das Tomographiebilddaten-Erzeugungsmodul (80, 580) ferner angeordnet ist zum Durchführen, für jedes Fotodetektorelement und für jede Wellenlänge, einer Bestimmung einer gemittelten Messung durch Mittelung der Vielzahl von Messungen, die durch das Fotodetektorelement für die Wellenlänge erfasst wurden, und zum Erzeugen der Tomographiebilddaten auf Grundlage der gemittelten Messung für jeden Fotodetektor und für jede Wellenlänge.

2. Konfokales Optische-Kohärenz-Tomographie-, OCT-, Abbildungssystem (10, 300, 400, 500, 800) zum Abbilden einer Region (20) eines Auges (30) einer Zielperson, wobei das konfokale OCT-Abbildungssystem (10, 300, 400, 500, 800) umfasst:

eine Lichtquelle (510, 810), die angeordnet ist zum Erzeugen von Licht;
ein Beleuchtungsmodul (50, 55), umfassend eine Maske (310) mit einer Vielzahl von durchlässigen Abschnitten (315), durch die sich das Licht von der Lichtquelle (510, 810) ausbreiten kann, um jeweilige nicht überlappende Strahlen (90) aus Licht zu bilden, wenn die Maske (310) von der Lichtquelle (510, 810) beleuchtet wird, wobei das Beleuchtungsmodul (50, 55) angeordnet ist zum Durchführen eines Abtastens der Region (20) des Auges (30) der Zielperson durch Abtasten der Vielzahl von nicht überlappenden Strahlen (90) über die Region (20), so dass jeder Punkt in der Region (20) während des Abtastens von einem entsprechenden der Vielzahl von nicht überlappenden Strahlen (90) beleuchtet wird;
ein Interferometer (60), umfassend einen Strahlteiler (320, 410), der angeordnet ist zum Erzeugen von Referenzlicht (322, 414) auf Grundlage des von der Lichtquelle (40, 510, 710, 810, 1010) erzeugten Lichts, wobei das Interferometer (60) angeordnet ist zum Erzeugen von Interferenzlicht (324, 445) durch Kombinieren, für jeden Strahl, von Licht von dem Strahl, der von der Region (20) reflektiert wurde, mit dem von dem Strahlteiler (320, 410) erzeugten Referenzlicht (322, 414);
eine Fotodetektorelement-Anordnung (70), die angeordnet ist zum Detektieren des Interferenzlichts (324, 445), das während des Abtastens erzeugt wird; und
ein Tomographiebilddaten-Erzeugungsmodul (80, 580), das angeordnet ist zum Erzeugen von Tomographiebilddaten (620, 630), die ein Tomographiebild der Region (20) auf Grundlage des detektierten Interferenzlichts (324, 445) definieren, wobei
das ophthalmische OCT-Abbildungssystem ein Schwenklichtquellen-OCT-Abbildungssystem (10, 300, 400, 500, 800) ist,
die Lichtquelle eine Schwenklichtquelle (510, 810) ist, die angeordnet ist zum Erzeugen von Licht mit variierender Wellenlänge,
das Beleuchtungsmodul (50, 55) angeordnet ist zum Durchführen einer Vielzahl von Abtastungen der Region (20) des Auges (30) der Zielperson, während die Wellenlänge des Lichts variiert wird, durch Abtasten der Vielzahl von sich nicht überlappenden Strahlen (90) über die Region (20), so dass jeder Punkt in der Region (20) durch einen entsprechenden Strahl der Vielzahl von sich nicht überlappenden Strahlen (90) während jeder der Abtastungen beleuchtet wird,
die Fotodetektorelemente (70) angeordnet sind zur Detektion des Interferenzlichtes (324), das während jeder der Abtastungen erzeugt wird,
**dadurch gekennzeichnet, dass**
die Integrationszeit jedes Fotodetektorelements (70) länger ist als die Dauer jeder Abtastung, so dass mehrere Abtastungen der Region (20) unter Verwendung von Licht der gleichen Wellenlänge durchgeführt wird, bevor eine Messung durch das Fotodetektorelement (70) erfasst wird.

3. Konfokales OCT-Abbildungssystem (10, 300, 500, 800) gemäß Anspruch 1 oder Anspruch 2, wobei das Interferometer (60) einen Referenzarm (330) aufweist, umfassend einen Referenzspiegel (335), und der Strahlteiler (320) angeordnet zum Extrahieren von Licht aus jedem der Strahlen (90) und zum Ausrichten des extrahierten Lichts auf den Referenzspiegel (335) ist, wobei der Spiegel (335) angeordnet zum Reflektieren des extrahierten Lichts zurück zum Strahlteiler (320) als das Referenzlicht (322), und wobei der Strahlteiler (320) angeordnet ist zum Erzeugen des Interferenzlichts (324) durch Kombinieren, für jeden Strahl, von Licht von dem Strahl, der von der Region (20) reflektiert wurde, mit dem Licht, das zurück zum Strahlteiler (320) durch den Spiegel (335) reflektiert wurde.

4. Konfokales OCT-Abbildungssystem (10, 400) gemäß Anspruch 1 oder Anspruch 2, wobei das Interferometer einen zweiten Strahlteiler (420) und einen Referenzarm (430) umfasst, entlang dessen das Referenzlicht (414) angeordnet zum Ausbreiten von dem Strahlteiler (410) zu dem zweiten Strahlteiler (420), wobei der zweite Strahlteiler (420) so angeordnet zum Erzeugen des Interferenzlichts (445) durch Kombinieren, für jeden Strahl, von Licht von dem Strahl, der von der Region (20) reflektiert worden ist, mit dem Referenzlicht (414), das sich entlang des Referenzarms (430) ausbreitet.

5. Konfokales OCT-Abbildungssystem (10, 300, 400, 500, 800) gemäß einem der vorhergehenden Ansprüche, wobei die Maske (310) ferner angeordnet zum Ermöglichen, dass das Licht, das von der Region (20) des Auges (30) der Zielperson reflektiert wurde, sich zu der Fotodetektorelement-Anordnung (70) nur durch die durchlässigen Abschnitte (315) der Maske (310) ausbreitet.

6.  Konfokales OCT-Abbildungssystem (10, 300, 400, 500, 800) gemäß einem der vorhergehenden Ansprüche, wobei die Lichtquelle eine Vollfeld-OCT-Lichtquelle (40, 510) ist, die angeordnet ist zum Bereitstellen einer Vollfeld-Beleuchtung für das Beleuchtungsmodul (50, 55).

7.  Konfokales OCT-Abbildungssystem gemäß einem der Ansprüche 1 bis 5, wobei die Lichtquelle eine Linienfeld-OCT-Lichtquelle (810) ist, die angeordnet ist zum Bereitstellen einer Lichtlinie für das Beleuchtungsmodul (50, 55), und wobei das Beleuchtungsmodul (50, 55) angeordnet ist zum Durchführen eines Abtastens der Region (20) des Auges (30) der Zielperson durch Abtasten der Vielzahl von nicht überlappenden Strahlen (90) über jede einer Vielzahl von Linien (910, 920), die die Region (20) kreuzen, so dass jeder Punkt in der Region (20) während des Abtastens von einem entsprechenden der Vielzahl von nicht überlappenden Strahlen (90) beleuchtet wird.

8.  Konfokales OCT-Abbildungssystem (10, 300, 400, 500, 800) gemäß einem der vorhergehenden Ansprüche, wobei die Maske (310) angeordnet ist zum Drehen während des Abtastens, und die durchlässigen Abschnitte (315) über die Maske (310) verteilt sind, so dass jeder Punkt in der Region (20) durch einen jeweiligen der Vielzahl von nicht überlappenden Strahlen (90) beleuchtet wird, wenn sich die Maske (310) während des Abtastens dreht.

9.  Konfokales OCT-Abbildungssystem (10, 300, 400, 500, 800) gemäß Anspruch 8, wobei die Vielzahl der durchlässigen Abschnitte (315) in einer archimedischen Spirale über die Maske (310) verteilt ist.

10. Konfokales OCT-Abbildungssystem (10, 300, 400, 500, 800) gemäß Anspruch 8 oder Anspruch 9, wobei das Beleuchtungsmodul (50, 55) ferner eine Vielzahl von Mikrolinsen (350) umfasst, wobei jede der Mikrolinsen (350) angeordnet ist zum Ausrichten von Licht von der Lichtquelle (40) durch einen jeweiligen der durchlässigen Abschnitte in der Maske (310), wobei die Vielzahl von Mikrolinsen (350) angeordnet ist zum gemeinsamen Drehen mit der Maske (310), um eine Ausrichtung jeder Mikrolinse (350) mit dem jeweiligen durchlässigen Abschnitt (315) während der Drehung der Maske (310) und der Vielzahl von Mikrolinsen (350) beizubehalten.

11. Konfokales OCT-Abbildungssystem (10, 300, 400, 500, 800) gemäß einem der Ansprüche 8 bis 10, wobei die durchlässigen Abschnitte (315) der Maske (310) mindestens eines von Nadellöchern oder Schlitzen in der Maske (310) umfassen.

**Revendications**

1.  Système d'imagerie (10, 300, 400, 500, 800) confocal par tomographie par cohérence optique, OCT, pour imager une région (20) d'un oeil (30) d'un sujet, le système d'imagerie (10, 300, 400, 500, 800) confocal par OCT comprenant :

    une source de lumière (510, 810) conçue pour générer de la lumière ;
    un module d'éclairage (50, 55) comprenant un masque (310) présentant une pluralité de parties transmettrices (315) à travers lesquelles la lumière provenant de la source de lumière (510, 810) peut se propager pour former des faisceaux (90) de lumière respectifs sans chevauchement lorsque le masque (310) est éclairé par la source de lumière (510, 810), le module d'éclairage (50, 55) étant conçu pour mettre en oeuvre un balayage de la région (20) de l'oeil (30) du sujet en balayant la pluralité de faisceaux (90) sans chevauchement à travers la région (20) de telle sorte que chaque point dans la région (20) est éclairé par un faisceau respectif de la pluralité de faisceaux (90) sans chevauchement pendant le balayage ;
    un interféromètre (60) comprenant un séparateur (320, 410) de faisceau conçu pour générer une lumière de référence (322, 414) sur la base de la lumière générée par la source de lumière (510, 810), l'interféromètre (60) étant conçu pour générer une lumière d'interférence (324, 445) en combinant, pour chaque faisceau, de la lumière provenant du faisceau qui a été réfléchi depuis la région (20) avec la lumière de référence (322, 414) générée par le séparateur (320, 410) de faisceau ;
    un réseau d'éléments photodétecteurs (70) conçus pour détecter la lumière d'interférence (324, 445) générée pendant le balayage ; et
    un module (80, 580) de génération de données d'image tomographique conçu pour générer des données (620, 630) d'image tomographique définissant une image tomographique de la région (20) sur la base de la lumière d'interférence (324, 445) détectée, dans lequel le système d'imagerie ophtalmique par OCT est un système d'imagerie (10, 300, 400, 500, 800) par OCT à source balayée,
    la source de lumière est une source de lumière (510, 810) balayée conçue pour générer de la lumière à longueur d'onde variable,

le module d'éclairage (50, 55) est conçu pour mettre en oeuvre une pluralité de balayages de la région (20) de l'œil (30) du sujet, tandis que la longueur d'onde de la lumière est en cours de variation, en balayant la pluralité de faisceaux (90) sans chevauchement à travers la région (20) de telle sorte que chaque point dans la région (20) est éclairé par un faisceau respectif de la pluralité de faisceaux (90) sans chevauchement pendant chacun des balayages,

les éléments photodétecteurs (70) sont conçus pour détecter la lumière d'interférence (324) générée pendant chacun des balayages,

**caractérisé en ce que**,

quand la longueur d'onde de la lumière délivrée en sortie par la source de lumière (510, 810) balayée est balayée à travers une pluralité de valeurs de longueur d'onde, le module d'éclairage (50, 55) est conçu pour mettre en oeuvre la pluralité de balayages de la région (20) en mettant en oeuvre une pluralité de balayages pour chaque longueur d'onde de lumière, dans lequel, pour chaque balayage, la superficie entière de la région (20) est éclairée par la pluralité de faisceaux (90) sans chevauchement de la longueur d'onde quand le masque (310) tourne,

et **en ce que**

chaque élément photodétecteur (70) du réseau est conçu pour détecter la lumière d'interférence (324) générée pendant chacun des balayages en générant, pour chaque longueur d'onde, une pluralité de mesures de la lumière d'interférence (324) correspondant à la pluralité de balayages mis en oeuvre à l'aide de lumière de cette longueur d'onde, et

**en ce que**

le module (80, 580) de génération de données d'image tomographique est en outre conçu pour mettre en oeuvre, pour chaque élément photodétecteur et pour chaque longueur d'onde, une détermination d'une mesure moyennée en faisant la moyenne de la pluralité de mesures obtenues par l'élément photodétecteur pour la longueur d'onde, et générer les données d'image tomographique sur la base de la mesure moyennée pour chaque photodétecteur et pour chaque longueur d'onde.

2. Système d'imagerie (10, 300, 400, 500, 800) confocal par tomographie par cohérence optique, OCT, pour imager une région (20) d'un oeil (30) d'un sujet, le système d'imagerie (10, 300, 400, 500, 800) confocal par OCT comprenant :

une source de lumière (510, 810) conçue pour générer de la lumière ;

un module d'éclairage (50, 55) comprenant un masque (310) présentant une pluralité de parties transmettrices (315) à travers lesquelles la lumière provenant de la source de lumière (510, 810) peut se propager pour former des faisceaux (90) sans chevauchement de lumière respectifs lorsque le masque (310) est éclairé par la source de lumière (510, 810), le module d'éclairage (50, 55) étant conçu pour mettre en oeuvre un balayage de la région (20) de l'œil (30) du sujet en balayant la pluralité de faisceaux (90) sans chevauchement à travers la région (20) de telle sorte que chaque point dans la région (20) est éclairé par un faisceau respectif de la pluralité de faisceaux (90) sans chevauchement pendant le balayage ;

un interféromètre (60) comprenant un séparateur (320, 410) de faisceau conçu pour générer une lumière de référence (322, 414) sur la base de la lumière générée par la source de lumière (40, 510, 710, 810, 1010), l'interféromètre (60) étant conçu pour générer une lumière d'interférence (324, 445) en combinant, pour chaque faisceau, de la lumière provenant du faisceau qui a été réfléchi depuis la région (20) avec la lumière de référence (322, 414) générée par le séparateur (320, 410) de faisceau ;

un réseau d'éléments photodétecteurs (70) conçus pour détecter la lumière d'interférence (324, 445) générée pendant le balayage ; et

un module (80, 580) de génération de données d'image tomographique conçu pour générer des données (620, 630) d'image tomographique définissant une image tomographique de la région (20) sur la base de la lumière d'interférence (324, 445) détectée, dans lequel

le système d'imagerie ophtalmique par OCT est un système d'imagerie (10, 300, 400, 500, 800) par OCT à source balayée,

la source de lumière est une source de lumière (510, 810) balayée conçue pour générer de la lumière à longueur d'onde variable,

le module d'éclairage (50, 55) est conçu pour mettre en oeuvre une pluralité de balayages de la région (20) de l'œil (30) du sujet, tandis que la longueur d'onde de la lumière est en cours de variation, en balayant la pluralité de faisceaux (90) sans chevauchement à travers la région (20) de telle sorte que chaque point dans la région (20) est éclairé par un faisceau respectif de la pluralité de faisceaux (90) sans chevauchement pendant chacun des balayages,

les éléments photodétecteurs (70) sont conçus pour détecter la lumière d'interférence (324) générée pendant chacun des balayages,
**caractérisé en ce que**
le temps d'intégration de chaque élément photodétecteur (70) est plus long que la durée de chaque balayage, de telle sorte que de multiples balayages de la région (20) sont mis en oeuvre à l'aide de lumière de la même longueur d'onde avant qu'une mesure ne soit acquise par l'élément photodétecteur (70).

3.  Système d'imagerie (10, 300, 500, 800) confocal par OCT selon la revendication 1 ou la revendication 2, dans lequel l'interféromètre (60) présente un bras de référence (330) comprenant un miroir (335) de référence, et le séparateur (320) de faisceau est conçu pour extraire de la lumière de chacun des faisceaux (90) et diriger la lumière extraite vers le miroir (335) de référence, dans lequel le miroir (335) est conçu pour réfléchir la lumière extraite de retour vers le séparateur (320) de faisceau en tant que lumière de référence (322), et dans lequel le séparateur (320) de faisceau est conçu pour générer la lumière d'interférence (324) en combinant, pour chaque faisceau, de la lumière provenant du faisceau qui a été réfléchi depuis la région (20) avec la lumière réfléchie de retour vers le séparateur (320) de faisceau par le miroir (335).

4.  Système d'imagerie confocal (10, 400) par OCT selon la revendication 1 ou la revendication 2, dans lequel l'interféromètre comprend un deuxième séparateur (420) de faisceau et un bras de référence (430) le long duquel la lumière de référence (414) est conçue pour se propager depuis le séparateur (410) de faisceau vers le deuxième séparateur (420) de faisceau, le deuxième séparateur (420) de faisceau étant conçu pour générer la lumière d'interférence (445) en combinant, pour chaque faisceau, de la lumière provenant du faisceau qui a été réfléchi depuis la région (20) avec la lumière de référence (414) se propageant le long du bras de référence (430).

5.  Système d'imagerie (10, 300, 400, 500, 800) confocal par OCT selon une quelconque revendication précédente, dans lequel le masque (310) est en outre conçu pour permettre à la lumière qui a été réfléchie depuis la région (20) de l'œil (30) du sujet de se propager vers le réseau d'éléments photodétecteurs (70) uniquement à travers les parties transmettrices (315) du masque (310).

6.  Système d'imagerie (10, 300, 400, 500, 800) confocal par OCT selon une quelconque revendication précédente, dans lequel la source de lumière est une source de lumière (40, 510) par OCT à champ complet conçue pour fournir un éclairage à champ complet au module d'éclairage (50, 55).

7.  Système d'imagerie confocal par OCT selon l'une quelconque des revendications 1 à 5, dans lequel la source de lumière est une source de lumière (810) par OCT à champ linéaire conçue pour fournir une ligne de lumière au module d'éclairage (50, 55), et dans lequel le module d'éclairage (50, 55) est conçu pour mettre en oeuvre un balayage de la région (20) de l'œil (30) du sujet en balayant la pluralité de faisceaux (90) sans chevauchement à travers chacune d'une pluralité de lignes (910, 920) traversant la région (20) de telle sorte que chaque point dans la région (20) est éclairé par un faisceau respectif de la pluralité de faisceaux (90) sans chevauchement pendant le balayage.

8.  Système d'imagerie (10, 300, 400, 500, 800) confocal par OCT selon une quelconque revendication précédente, dans lequel le masque (310) est conçu pour tourner pendant le balayage, et les parties transmettrices (315) sont réparties à travers le masque (310) de telle sorte que chaque point dans la région (20) est éclairé par un faisceau respectif de la pluralité de faisceaux (90) sans chevauchement quand le masque (310) tourne pendant le balayage.

9.  Système d'imagerie (10, 300, 400, 500, 800) confocal par OCT selon la revendication 8, dans lequel la pluralité de parties transmettrices (315) sont réparties à travers le masque (310) en une spirale d'Archimède.

10. Système d'imagerie (10, 300, 400, 500, 800) confocal par OCT selon la revendication 8 ou la revendication 9, dans lequel le module d'éclairage (50, 55) comprend en outre une pluralité de micro-lentilles (350), chacune des micro-lentilles (350) étant conçue pour diriger la lumière provenant de la source (40) de lumière à travers une partie respective des parties transmettrices dans le masque (310), dans lequel la pluralité de micro-lentilles (350) sont conçues pour tourner avec le masque (310) de manière à maintenir un alignement de chaque micro-lentille (350) avec la partie transmettrice (315) respective pendant la rotation du masque (310) et la pluralité de micro-lentilles (350).

11. Système d'imagerie (10, 300, 400, 500, 800) confocal par OCT selon l'une quelconque des revendications 8 à 10, dans lequel les parties transmettrice (315) du masque (310) comprennent au moins un parmi des trous d'épingle ou des fentes dans le masque (310).

Fig. 1

Fig. 2

Fig. 3

Fig. 4

Fig. 5

Fig. 6A

Fig. 6B

Fig. 7

Fig. 8

Fig. 9

Fig. 10

Fig. 11

**EP 3 977 915 B1**

## REFERENCES CITED IN THE DESCRIPTION

**Patent documents cited in the description**

- WO 2014089504 A1
- JP 2009008393 A
- US 2007263226 A1